(19) Europäisches Patentamt / European Patent Office / Office européen des brevets

(11) **EP 4 515 551 B1**

(12) **EUROPÄISCHE PATENTSCHRIFT**

(45) Veröffentlichungstag und Bekanntmachung des Hinweises auf die Patenterteilung:
**09.04.2025   Patentblatt 2025/15**

(21) Anmeldenummer: **23805620.4**

(22) Anmeldetag: **16.11.2023**

(51) Internationale Patentklassifikation (IPC):
**G16C 20/20** (2019.01)

(52) Gemeinsame Patentklassifikation (CPC):
**G16C 20/20;** G16C 20/70

(86) Internationale Anmeldenummer:
**PCT/EP2023/081994**

(87) Internationale Veröffentlichungsnummer:
**WO 2024/110291 (30.05.2024 Gazette 2024/22)**

(54) **BESTIMMUNG DER STABILITÄT EINES STOFFS ODER STOFFGEMISCHS**

DETERMINING THE STABILITY OF A SUBSTANCE OR SUBSTANCE MIXTURE

DÉTERMINATION DE LA STABILITÉ D'UNE SUBSTANCE OU D'UN MÉLANGE DE SUBSTANCES

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **21.11.2022   PCT/EP2022/082679**

(43) Veröffentlichungstag der Anmeldung:
**05.03.2025   Patentblatt 2025/10**

(73) Patentinhaber: **Bionorica SE**
**92318 Neumarkt (DE)**

(72) Erfinder:
• **DITTMER, Martin**
**93053 Neumarkt (DE)**
• **WIESNETH, Stefan**
**93053 Regensburg (DE)**

(74) Vertreter: **Best, Bastian**
**Bestpatent**
**Patentanwalt Bastian Best**
**Konrad-Zuse-Platz 8**
**81829 München (DE)**

(56) Entgegenhaltungen:
• **FENG LEI ET AL: "Nondestructive Detection of Postharvest Quality of Cherry Tomatoes Using a Portable NIR Spectrometer and Chemometric Algorithms", FOOD ANALYTICAL METHODS, SPRINGER NEW YORK LLC, US, vol. 12, no. 4, 8 January 2019 (2019-01-08), pages 914 - 925, XP036743020, ISSN: 1936-9751, [retrieved on 20190108], DOI: 10.1007/S12161-018-01429-9**
• **PEYVASTEH MOTAHAREH ET AL: "Meat freshness revealed by visible to near-infrared spectroscopy and principal component analysis", JOURNAL OF PHYSICS COMMUNICATIONS, vol. 4, no. 9, 1 September 2020 (2020-09-01), pages 095011, XP093126110, ISSN: 2399-6528, Retrieved from the Internet <URL:https://iopscience.iop.org/article/10.1088/2399-6528/abb322> DOI: 10.1088/2399-6528/abb322**
• **JOHNSTON CAROL S ET AL: "Stability of ascorbic acid in commercially available orange juices", J AM DIET ASSOC., vol. 102, no. 4, 1 April 2022 (2022-04-01), pages 525 - 529, XP093126338, DOI: 10.1016/s0002-8223(02)90119-7**
• **ROSA M ALONSO-SALCES ET AL: "H-NMR fingerprinting to evaluate the stability of olive oil", FOOD CONTROL, BUTTERWORTH, LONDON, GB, vol. 22, no. 12, 26 May 2011 (2011-05-26), pages 2041 - 2046, XP028100708, ISSN: 0956-7135, [retrieved on 20110606], DOI: 10.1016/J.FOODCONT.2011.05.026**

- **QIN YUHUA ET AL: "Similarity measure method based on spectra subspace and locally linear embedding algorithm", INFRARED PHYSICS AND TECHNOLOGY, ELSEVIER SCIENCE, GB, vol. 100, 13 May 2019 (2019-05-13), pages 57 - 61, XP085788686, ISSN: 1350-4495, [retrieved on 20190513], DOI: 10.1016/ J.INFRARED.2019.05.006**
- **POSADA H ET AL: "Stability across environments of the coffee variety near infrared spectral signature", HEREDITY, vol. 102, no. 2, 29 October 2008 (2008-10-29), Cham, pages 113 - 119, XP093126117, ISSN: 0018-067X, Retrieved from the Internet <URL:http://www.nature.com/ articles/hdy200888> DOI: 10.1038/hdy.2008.88**
- **MOROZOVA MARIA ET AL: "DISCRIMINANT ANALYSIS AND MAHALANOBIS DISTANCE (NIR DIFFUSE REFLECTANCE SPECTRA) IN THE ASSESSMENT OF DRUG'S BATCH-TO-BATCH DISPERSION AND QUALITY THRESHOLD ESTABLISHMENT", EUROPEAN SCIENTIFIC JOURNAL, ESJ, vol. 9, no. 27, 1 September 2013 (2013-09-01), pages 8 - 25, XP093126122, ISSN: 1857-7881**

**Beschreibung**

## TECHNISCHES GEBIET

Die vorliegende Erfindung betrifft allgemein das technische Gebiet der Analytik, insbesondere der pharmazeutischen Analytik.

## HINTERGRUND DER ERFINDUNG

Die Entwicklung neuer Produkte, beispielsweise im Arzneimittel-, Kosmetika- und Lebensmittelbereich, unterliegt einem immer größeren Anforderungskatalog und strengen Qualitätskriterien. Bei solchen Entwicklungen spielen insbesondere Stabilitätsuntersuchungen eine große Rolle, welche eine Vielzahl von möglichen Einflussfaktoren abdecken sollen (beispielsweise chemische oder physikalische Inkompatibilitäten zwischen Molekülen in einem Produkt, Belastbarkeit bzgl. Temperatur und/oder Luftfeuchtigkeit, Beständigkeit gegen UV/VIS-Strahlung, Oxidation, Interaktionen zwischen unterschiedlichen Reaktanden, mikrobieller Befall, biologischer Abbau usw.).

Die Prüfung jedes Parameters in einem Produkt individuell auf seine Reaktionen auf die genannten und weitere Faktoren kann nach etabliertem Ansatz nur durch eine große Anzahl verschiedener technischer Methoden charakterisiert werden. So bedarf es typischerweise mehrerer verschiedener HPLC-Methoden ("High Performance Liquid Chromatography"; (Hochleistungsflüssigkeitschromatographie)) für unterschiedliche Analyten, nasschemische kolorimetrische Gruppenreaktionen, Feuchtigkeitsbestimmung sowie dem Test physikalischer Parameter (beispielsweise Bruchfestigkeit, Friabilität und Zerfallszeit von Tabletten; Fließfähigkeit, Schütt- und Stampfdichte von Pulvern; Trübungsmessungen von Flüssigkeiten; und viele mehr).

Um möglichst viele solcher potenziellen Interaktionen abbilden zu können, ist es übliche Praxis, die Projektplanung durch ein "Design of Experiments" (DoE, Synonym: Statistische Versuchsplanung) zu planen. Dieses berücksichtigt in der Regel nicht nur alle möglichen qualitativen Zusammensetzungen, sondern auch quantitative Verhältnisse enthaltener Stoffe. Zusätzlich kann beispielsweise auch noch in Betracht gezogen werden, durch welches Packmittel (Primär- und/oder Sekundär- oder weitere Packmittel) ein Produkt am besten vor Veränderung geschützt werden kann. Als Packmittel werden Erzeugnisse bezeichnet, die zur Verpackung von Produkten verwendet werden. Sollen alle diese Aspekte auch in einen Versuchsplan integriert werden, multipliziert sich nochmals die Anzahl an Produktvarianten mit den zu testenden Packmittel-Optionen.

Unterschieden werden gemeinhin Primärpackmittel und Sekundärpackmittel. Primärpackmittel für Arzneimittel und Medizinprodukte umfassen Behältnisse bzw. Komponenten aus Glas, Gummi, Kunststoff, Aluminium und Verbundwerkstoffen sowie Folien. Diese Materialien kommen unmittelbar mit den medizinischen Produkten in Berührung und müssen daher bestimmte Anforderungen hinsichtlich Sicherheit, Wirksamkeit und Zuverlässigkeit erfüllen. Die Hersteller von Primärpackmitteln müssen den Erwartungen der Arzneimittelhersteller entsprechen und nachweisen können, dass ihre Produktionsprozesse einem integrierten Qualitätsmanagementsystem (QMS) sowie den Regeln der Guten Herstellungspraxis (Good Manufacturing Practice, GMP) unterliegen und somit den geforderten Qualitätsstandards genügen. Sekundärpackmittel sind als Umverpackungen zu verstehen, die nicht in direktem Kontakt zu den zu verpackenden Gegenständen bzw. Arzneimitteln oder anderen Stoffen stehen und die meist Schutz- und Kontrollfunktion haben.

Auf diese Weise ergibt sich ein Projektdesign mit einer großen Probenzahl gepaart mit zahlreichen Analysemethoden, die im Idealfall lückenlos für alle Proben durchgeführt werden müssten. Da jedoch ein enormer Informationsgewinn durch derartige Versuchsdesigns zu erwarten ist, ist eine Durchführung wünschenswert, um am Ende das bestmögliche Produkt mit der längsten Produktlaufzeit zu ermitteln. Dementgegen steht die Notwendigkeit eines sehr großen Personal- und Geräteaufwands, der für die Umsetzung mit klassischen analytischen Methoden erforderlich wäre. Mit dem etablierten Personalstamm und Gerätefuhrpark könnte eine so umfängliche Untersuchung nur unter großen Schwierigkeiten durchgeführt werden, da die dann erforderliche Arbeitszeit sowie die finanziellen Investitionen kaum akzeptabel wäre, und sich Timelines für derartige Produktentwicklungen massiv strecken würden. Die Alternative wäre eine drastische Reduktion des Messaufwands, die jedoch zu inakzeptabler Produktqualität führen könnte.

Neben dem enormen Aufwand für das Erheben eines umfänglichen Datensatzes im Rahmen einer Stabilitätsstudie stellt auch die Interpretation eines solchen gewaltigen Datensatzes eine große Herausforderung dar. Diese begründet sich darin, dass bei der "klassischen" Betrachtung der Originaldaten, bei der häufig nur eine kleine Teilmenge der Gesamtdaten ausgewertet wird (beispielsweise Betrachtung nur der drei stärksten Signale eines HPLC-Chromatogramms statt der Betrachtung des kompletten "Fingerprint-Bereichs") ein schwacher Trend oder eine Auffälligkeit unter Umständen maskiert oder gar nicht erfasst wird. Abhilfe können hier grundsätzlich moderne chemometrische Methoden liefern. Diese umfassen beispielsweise Hauptkomponentenanalysen, Korrelationsanalysen, Distanzmaße, Partial Least Squares Regression, Support Vector Machines, neuronale Netze. Derartige Methoden sind in der Lage, einen komplexen Originaldatensatz mit zahlreichen Variablen (hier: Prüfparameter) in wenige, latente Variablen umzurechnen, die jedoch immer noch in der Lage sind, den Datensatz mit ausreichender Genauigkeit zu beschreiben. Daher ist auch die Implementierung chemometrischer Methoden zur einfachen und effizienten Auswertung eines Datensatzes für die Lösung des Problems unbedingt erforderlich. Aufgrund der oben genannten personellen, finanziellen sowie zeitlichen Aspekte, werden Produkte derzeit nur hinsichtlich weniger Parameter auf ihre Stabilität hin geprüft. Dies führt dazu, dass bestimmte andere nicht gemessene Parameter, die die Stabilität beeinflussen können, entweder nicht gefunden werden und auf diese Weise zu einem minderwertigen Produkt führen können. Es ist daher von immensem Vorteil, die Eigenschaften eines Produkts hinsichtlich der Stabilität in seiner Gesamtheit zu erfassen und nicht anhand einzelner ausgewählter Parameter, die die Stabilität eines Stoffs oder Stoffgemischs, aus dem das Produkt oder z.B. dessen Verpackung besteht, in seiner Gesamtheit möglicherweise nicht korrekt reflektieren. Wichtige Parameter könnten so außer Betracht bleiben und zu nicht optimalen Produkten führen.

Der wissenschaftliche Artikel "Nondestructive Detection of Postharvest Quality of Cherry Tomatoes Using a Portable NIR Spectrometer and Chemometric Algorithms" von Lei Feng et al. (Food Analytical Methods (2019), DOI: 10.1007/s12161-018-01429-9) beschreibt eine Studie zur Eignung von chemometrischen Algorithmen beim Erkennen der Qualität von Cherrytomaten nach der Ernte. Die Studie vergleicht die Ergebnisse verschiedener Algorithmen und Daten-Vorverarbeitungstechniken.

Die vorliegende Erfindung löst die oben skizzierten Probleme, indem sie die Eigenschaften eines Produkts bzw. Stoffs oder Stoffgemischs (nachfolgend "Stoffgemisch") in ihrer Gesamtheit erfasst und bezüglich der Stabilität des gesamtem Produkts eine konkrete Aussage ermöglicht. Die Analyse einer Vielzahl von Parametern ist demzufolge aufgrund der vorliegenden Erfindung nicht mehr notwendig.

Es ist deshalb eine Aufgabe der vorliegenden Erfindung, ein Verfahren zur Bestimmung der Stabilität eines Stoffgemischs bereitzustellen, mit dem die oben genannten Nachteile des Standes der Technik überwunden werden können. Weiterhin kann das Berechnungsverfahren auch zur Überwachung von Prozessen, bei denen eine Vielzahl von Überwachungsparametern parallel erfasst werden, verwendet werden.

## BESCHREIBUNG

Die Erfindung wird in den Ansprüchen definiert.

Die vorliegende Erfindung umfasst ein computerimplementiertes Verfahren zur Bestimmung der Stabilität eines Stoffgemischs, sowie für die Überwachung von Prozessen, bei denen eine Vielzahl von Überwachungsparametern parallel erfasst werden. Das Verfahren umfasst mindestens einen Datenerfassungsschritt, in dem zumindest ein Messdatensatz empfangen wird, sowie mindestens einen zeitlich nachfolgenden Datenerfassungsschritt, in dem mindestens ein weiterer Messdatensatz empfangen wird. Der erste Datenerfassungsschritt umfasst den mindestens einen Startwert-Messdatensatz. Jeder Messdatensatz kann ein chemisches, insbesondere phytochemisches, Profil eines jeweiligen Stoffgemischs repräsentieren. Das Verfahren kann ferner einen Datenauswertungsschritt umfassen. Eine Auswertung ist die Verarbeitung von (Roh-)Daten eines Experiments mit den (Roh-)Daten aus dem mindestens einen Startwert-Messdatensatz und dem mindestens einen weiteren Messdatensatz zur Generierung eines konkreten Wissensgewinns. Der Datenauswertungsschritt umfasst für jeden Messdatensatz ein Bestimmen eines Startwerts des jeweiligen Stoffgemischs auf Basis des Messdatensatzes. Ferner umfasst der Datenauswertungsschritt ein Quantifizieren der Veränderung des jeweiligen Stoffgemischs in Bezug auf den Startwert mittels eines mathematischen Distanzmaßes durch Erfassen von mindestens einem weiteren, zeitlich nachfolgenden Datenerfassungsschritt, mit dem mindestens ein weitere Messdatensatz erfasst wird. Das Verfahren kann weiterhin einen Datenausgabeschritt umfassen, in dem für jeden Messdatensatz die Veränderung des jeweiligen Stoffgemischs grafisch dargestellt wird.

Ein Stoffgemisch nach der vorliegenden Erfindung umfasst sowohl einzelne Stoffe als auch Kombinationen oder Stoffgemische sowie chemische und/oder biologische Produkte. Die Begriffe "Stoff oder Stoffgemisch", "Kombination" und "Produkt" werden im Rahmen der vorliegenden Erfindung als äquivalente Begriffe verstanden.

Als die Stabilität eines Stoffgemischs im Sinne der vorliegenden Erfindung wird ein Maß für den Zeitraum verstanden, in der die Veränderung des jeweiligen Stoffgemischs innerhalb vorher definierter Grenzwerte bleibt. Dabei ist insbesondere die chemische und/oder die physikalische Stabilität umfasst. Im Rahmen einer Stabilitätsstudie kann die Stabilität entsprechend dem erfindungsgemäßen Verfahren untersucht werden. Eine Stabilitätsstudie wird daher als Experiment verstanden, mit dem untersucht wird, ob verschiedene Stoffgemische überhaupt stabil sind, bzw. wie schnell die Stabilität abnimmt. Alternativ oder zusätzlich kann vorgesehen sein, dass im Rahmen einer derartigen Untersuchung verschiedene Stoffgemische, insbesondere Formulierungen, untereinander verglichen werden. Die Stabilität beinhaltet die Veränderung eines Stoffs oder Stoffgemisches über einen Zeitraum, der zwischen mindestens zwei Zeitpunkten gemessen wird. Der erste Zeitpunkt umfasst den ersten Datenerfassungsschritt mit dem mindestens einen Startwert-Messdatensatz, der zweite umfasst den zweiten und alle weiteren Datenerfassungsschritte mit dem mindestens einen weiteren Messdatensatz.

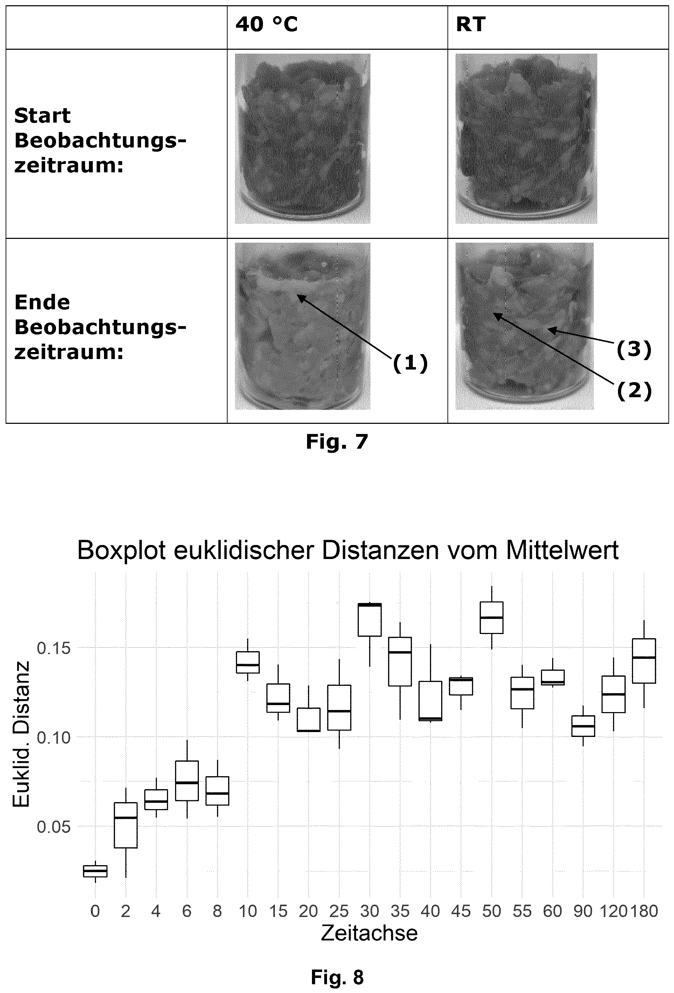

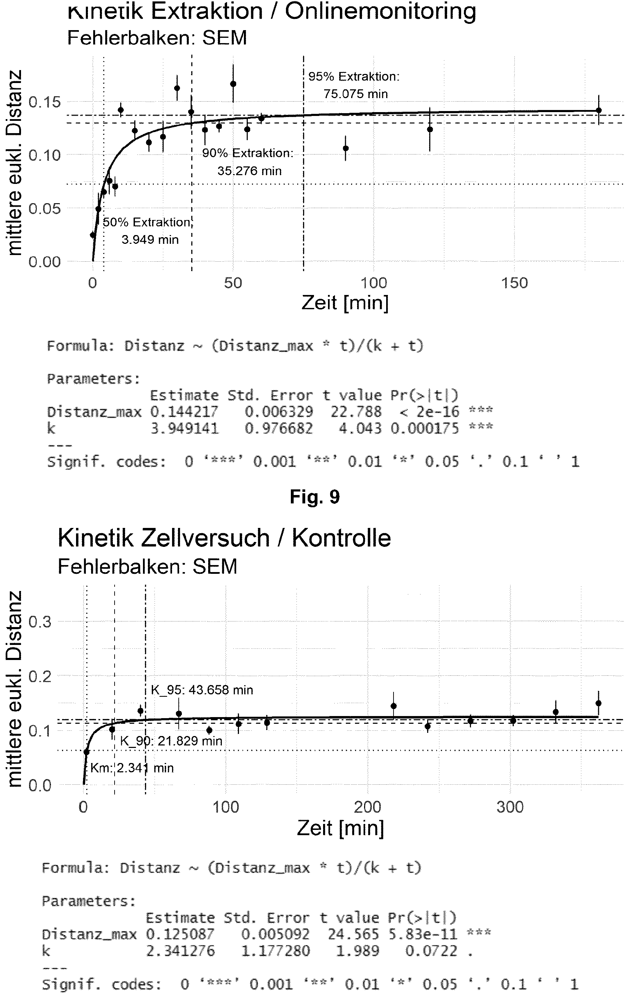

Weiter kann dieses Verfahren auch dazu genutzt werden, Produktionsprozesse oder Reaktionen zu überwachen (siehe Anwendungsbeispiel 3). Diese umfassen chemische oder biologische Produktionsprozesse und Reaktionen, beispielsweise Extraktionen, Tablettierungen, Kapselabfüllungen, Mischprozesse. So kann beispielsweise eine Gleichgewichtseinstellung in einem Extraktionsprozess ohne vorherige Kalibrierung einer Methode durch das neuartige Verfahren überwacht und der Zeitpunkt der Gleichgewichtseinstellung festgestellt werden. Die Gleichgewichtseinstellung wird beim vorliegenden Verfahren durch asymptotische Näherung der errechneten Distanzwerte an ein Plateau angezeigt, wie dargestellt in Fig. 8 und 9.

Ein chemisches Profil eines Stoffgemischs ist als Gesamtheit der chemischen Verbindungen des Stoffgemischs und/oder der dadurch begründeten chemischen Eigenschaften oder Reaktivität des Stoffgemischs zu verstehen. Ein phytochemisches Profil eines Stoffgemischs wird als Gesamtheit der aus pflanzlichen Bestandteilen stammenden chemischen Verbindungen des Stoffgemischs und/oder der dadurch begründeten Eigenschaften des Stoffgemischs verstanden.

Die oben beschriebenen Herausforderungen und Anforderungen definieren die Kriterien, welche ein neuartiges analytisches Konzept erfüllen muss. Das erfindungsgemäße Verfahren ermöglicht eine schnelle und umfangreiche Untersuchung von parallel angesetzten Stoffgemischen und deren Vergleich untereinander, auch über einen zeitlichen Verlauf hinweg. Somit wird in vorteilhafter Weise ein relativer Vergleich von Produktvarianten ermöglicht, um so eine Auswahl vielversprechender Kandidaten zu selektieren, beispielsweise im Rahmen der Arzneimittelforschung.

Im Rahmen der vorliegenden Offenbarung sei unter einem Arzneimittel verstanden: a) Alle Stoffe oder Stoffzusammensetzungen/-gemische, die als Mittel mit Eigenschaften zur Heilung oder zur Verhütung menschlicher Krankheiten bzw. Tierkrankheiten bestimmt sind, oder b) alle Stoffe oder Stoffzusammensetzungen/-gemische, die im oder am menschlichen bzw. tierischen Körper verwendet oder einem Menschen bzw. Tier verabreicht werden können, um entweder die menschlichen bzw. tierischen physiologischen Funktionen durch eine pharmakologische, immunologische oder metabolische Wirkung wiederherzustellen, zu korrigieren oder zu beeinflussen oder eine medizinische Diagnose zu erstellen.

Ein besonderer Vorteil, der dem erfindungsgemäßen Verfahren zugrunde liegt, ist es, dass mit einem sehr geringen Aufwand während einer Produktentwicklung eine deutlich reduzierte Auswahl möglicher Produkt-Kandidaten generiert werden kann, so dass anschließend beispielsweise nur diese Produkt-Kandidaten durch weitere dafür spezifische klassische analytische Methoden weiter untersucht werden müssen. Durch die Konzentration auf bestimmte vielversprechende Produkt-Kandidaten kann weiteres Detailwissen zur Ermöglichung einer finalen Auswahl der besten Produktvariante effizient erlangt werden. Insbesondere kann durch das vorgeschlagene erfindungsgemäße Verfahren die analytische Bearbeitung groß angelegter Produktvergleiche mit sehr geringen Personal- und Gerätekapazitäten in angemessener Zeit möglich gemacht werden. Das erfindungsgemäße Verfahren ist besonders vorteilhaft anwendbar für komplexe Stoffgemische, ist jedoch auch einsetzbar für Produkte mit wenigen Einzelkomponenten sowie Einzelverbindungen.

Das erfindungsgemäße Verfahren ermöglicht es, durch eine umfassende Auswertung komplexer Messdatensätze, die ein möglichst vollumfängliches (phyto-) chemisches Profil aller Proben einer Stabilitätsuntersuchung repräsentieren, diejenigen Stoffgemische zu identifizieren, welche die geringste Veränderung im Verhältnis zu deren Startwert und geringste Varianz zeigen und somit als am stabilsten angenommen werden können.

Vorzugsweise ist vorgesehen, dass das Verfahren, insbesondere der Datenauswertungsschritt, das Verwenden eines maschinellen Lernmodells ("machine learning model") umfasst, welches vorzugsweise durch unüberwachtes ("unsupervised") und/oder überwachtes maschinelles Lernen ("supervised machine learning") erzeugt bzw. trainiert wurde. Unsupervised machine learning ist ein mathematisches oder informationstechnisches Verfahren, bei dem eine Datenverarbeitungseinrichtung / Computer ohne externe Zusatzinformationen Daten verarbeitet und selbständig eine Lösung zu finden versucht. Ein Beispiel hierfür ist die "Principal component analysis" (Hauptkomponentenanalyse, Abk: PCA). Supervised machine learning ist ein mathematisches oder informationstechnisches Verfahren, bei dem eine Datenverarbeitungseinrichtung / Computer mit Hilfe externer Informationen (beispielsweise Trainingsdaten, insbesondere Informationen über Konzentrationen, die in einem Experiment verwendet wurden) verarbeitet. Eine selbstständige Lösungssuche erfolgt dabei typischerweise nach Kalibration auf bereits bekannte Daten.

Besonders bevorzugt ist vorgesehen, dass der zumindest eine Messdatensatz mittels Nahinfrarotspektroskopie erhaltene Daten umfasst.

Durch eine Nahinfrarotspektroskopie (NIR) können Proben von Stoffgemischen ohne komplizierte Probenaufarbeitung und zerstörungsfrei gemessen werden, um Messdatensätze zu erzeugen. Nahinfrarotspektroskopie, NIR-Spektroskopie oder NIRS abgekürzt, ist eine physikalische Analysentechnik auf Basis der Spektroskopie im Bereich des kurzwelligen Infrarotlichts. Bei der NIRS findet die Detektion im nahen Infrarot vorzugsweise von ca. 760-2500 nm (umgerechnet ca. 13160 $cm^{-1}$ bis 4000 $cm^{-1}$) statt. Diese Technologie erfasst Kombinations- und Obertonschwingungen aller Moleküle einer Probe und ist daher in der Lage, diese ganzheitlich zu erfassen. Um diese Daten zu verwerten, kommen insbesondere mathematische Verfahren (Chemometrie) zum Einsatz, da die enthaltenen Informationen (beispielsweise An-/ Abwesenheit bestimmter chemischer Verbindungen, Konzentrationen etc.) dieser Spektren dem Betrachter sonst in der Regel verborgen bleiben. Die Nahinfrarotspektroskopie kann in vorteilhafter Weise gleichermaßen für flüssige Proben (Messung beispielsweise von Transmission, d.h. der Messstrahl durchquert die Probe in einer definierten Schichtdicke und das nicht absorbierte Licht wird durch einen Detektor erfasst oder Transflexion, d.h. der Messstrahl trifft auf die Probe. Das zurückgeworfene Licht wird von einem Detektor erfasst. Die Absorption gibt die Differenz aus eingestrahltem und reflektiertem Licht an) sowie für feste Proben (Messung beispielsweise diffuser Reflexion oder Transmission) Anwendung finden.

Ein Aspekt des erfindungsgemäßen Verfahrens ist es, eine maschinelle und maschinell reproduzierbare Datenauswertung von Messdatensätzen bereitzustellen. Der Vorteil von Datensätzen, die beispielsweise mittels einer Nahinfrarotspektroskopie gewonnen werden besteht darin, dass diese Datensätze - verglichen mit Datensätzen, die aus anderen analytischen Techniken gewonnen werden - sehr viel mehr Informationen in einem Datum einer Beobachtung enthalten, denn klassische analytische Techniken beobachten nur wenige isolierte Signale gleichzeitig. Die Nahinfrarotspektroskopie ermöglicht eine breitere Auswertung einer Vielzahl von Signalen.

Vorzugsweise ist vorgesehen, dass der zumindest ein Messdatensatz zusätzlich oder alternativ umfasst: mittels UV/VIS-Spektroskopie erhaltene Daten, mittels Raman-Spektroskopie erhaltene Daten, einen (U)HPLC-Fingerprint, einen GC-Fingerprint, einen Peaktable aus einem chromatographischen Verfahren, und/oder zumindest einen physikalischen, biologischen oder chemischen Parameter, insbesondere Zuckergehalt, Zerfallsgeschwindigkeit, Farbe, Bruchfestigkeit, Zerfallszeit, Friabilität, Dichte, Viskosität, Brechungsindex und/oder optischer Drehwinkel.

UV/VIS-Photometrie bedeutet ein zur optischen Molekülspektroskopie gehörendes spektroskopisches Verfahren, das elektromagnetische Wellen des ultravioletten (UV) und des sichtbaren (englisch visible, VIS) Lichts nutzt, wobei eine Lichtquelle ultraviolettes und sichtbares im Wellenlängenbereich von etwa 200 nm bis ca. 800 nm ausstrahlt.

Bei der Raman-Spektroskopie wird die zu untersuchende Materie mit monochromatischem Licht bestrahlt, vorzugsweise mit einem Laser. Im Spektrum des an der Probe gestreuten Lichts werden neben der eingestrahlten Frequenz (Rayleigh-Streuung) weitere Frequenzen beobachtet. Die Frequenzunterschiede zum eingestrahlten Licht entsprechen den für das Material charakteristischen Energien von Rotations-, Schwingungs-, Phonon- oder Spin-Flip-Prozessen. Aus dem erhaltenen Spektrum lassen sich Rückschlüsse auf die untersuchte Substanz ziehen.

(U)HPLC ((Ultra) high performance liquid chromatography) ist eine analytische StandardMethode (Flüssigchromatographie-Verfahren), mit dem man nicht nur Substanzen trennt, sondern diese auch über Standards identifizieren und quantifizieren kann. Mittels (U)HPLC können auch nicht-flüchtige Substanzen analysiert werden. Diese kann an ein sensitives Detektor-System wie Massenspektrometrie (MS oder zusammen auch UHPLC MS/MS) angeschlossen werden.

Im Rahmen der Analysen ist die vollständige Aufzeichnung der Ergebnisse als Muster ("fingerprint") möglich. Diese "fingerprints" können mit denen einer Datenbank oder Ergebnissen aus anderen Analysen mit derselben Methode verglichen werden, um die analysierte Substanz zu identifizieren.

Das erfindungsgemäße Verfahren ermöglicht es in vorteilhafter Weise, verschiedene analytische Datensätze zu kombinieren und in eine Auswertung einfließen zu lassen. So können die zumindest einen Messdatensätze zu einem Stoffgemisch beispielsweise Datensätze umfassen, die mittels UV/VIS-Spektroskopie, Raman-Spektroskopie, (U) HPLC-Fingerprints (insbesondere aus einer Kopplung mit unterschiedlichen Detektoren, zum Beispiel Diodenarray-Detektor (DAD), Brechungsindex-Detektor, Elektrochemischer-Detektor, UV/VIS-Detektor, Massenspektrometrie-Detektor (MS), Lichtstreudetektor (ELSD, engl. Evaporative Light Scattering Detector), GC-Fingerprints (insbesondere aus einer Kopplung mit diversen Detektoren, zum Beispiel Flammenionisationsdetektor (FID), MS) gewonnen wurden oder ganz generell Peaktables aus allen denkbaren chromatographischen Verfahren umfassen. Das erfindungsgemäße Verfahren kann mit anderen Worten auf eine Vielzahl von Messtechniken angewendet werden, die ein oder mehrere X-Werte (beispielsweise Wellenlängen, m/z-Verhältnisse (Masse/Ladungsverhältnis), Retentionszeiten, Messpunkte) ein oder mehreren Y-Werte (beispielsweise Intensitäten, Absorptionen, Spannungen, Messwerte) zuordnen. Das Verfahren ist somit besonders vielseitig und flexibel einsetzbar.

Darüber hinaus können bei entsprechenden Analysenmethoden (beispielsweise HPLC-DAD, HPLC-MS) auch mehrdimensionale Datensätze verarbeitet werden. Weiterhin können auch große Datenmatrices aus individuell (d. h. auch mit verschiedenen Techniken) erhobenen Prüfparametern (beispielsweise physikalisch, biologisch oder chemische Analysen (ohne darauf beschränkt zu sein) zu einer Auswertung kombiniert und dem neuen Verfahren unterworfen werden. Beispielsweise könnten so die Messung des Zuckergehalts, die Zerfallsgeschwindigkeit, die Farbe der Tablette und die Bruchfestigkeit zu einem Datensatz, der über mehrere Zeitpunkte gemessen wurde zu einer Datenmatrix zusammengestellt und ausgewertet werden.

Es kann ferner vorgesehen sein, dass die Quantifizierung der Veränderung des jeweiligen Stoffgemischs auf der Gesamtheit der Inhaltsstoffe des jeweiligen Stoffgemischs basiert.

Durch eine Berücksichtigung der Gesamtheit aller Inhaltsstoffe eines zu untersuchenden Stoffgemischs wird die Generierung belastbarer Ergebnisse in besonders einfacher Weise ermöglicht. Es werden insbesondere alle spektralen Veränderungen aller Inhaltsstoffe in Bezug auf einen Startwert bei einer Stabilitätsuntersuchung berücksichtigt. Anders als bei der klassischen Markeranalytik (beispielsweise mittels HPLC) wird hierdurch die ganzheitliche Betrachtung aller Inhaltsstoffe komplexer Gemische (beispielsweise mittels Nahinfrarotspektroskopie) ermöglicht, wodurch die grundlegende Veränderung der Gesamtprobe abgebildet wird.

Die Veränderung des Stoffgemischs kann durch die Verwendung eines mathematischen Distanzmaß berechnet werden.

Vorzugsweise ist vorgesehen, dass das mathematische Distanzmaß ausgewählt ist aus der folgenden Gruppe: euklidische Distanz, Mahalanobis-Distanz, Manhattan-Distanz, Pearson-Distanz und/oder Gower-Distanz.

Durch die Auswahl eines mathematischen Distanzmaßes aus der oben erwähnten Gruppe wird gewährleistet, dass die Veränderung eines zu untersuchenden Stoffgemischs in einem zeitlichen Verlauf quantifiziert wird, wobei die Komplexität der zugrundeliegenden Datensätze ganzheitlich verarbeitet werden kann. Die Wahl einer geeigneten mathematische Strategie, den Grad der Veränderung einer Probe in einem zeitlichen Verlauf zu quantifizieren, ermöglicht somit eine effiziente Quantifizierung der Veränderung eines jeweiligen Stoffgemischs. Es wird insbesondere ein sehr guter vergleichender Überblick über einen Datensatz einer Stabilitätsuntersuchung gewonnen, ohne eine Vielzahl separater Parameter einzeln prüfen zu müssen.

Besonders vorteilhaft ist vorgesehen, dass das mathematische Distanzmaß durch einen Benutzer auswählbar ist.

Durch die Möglichkeit, dass ein Benutzer ein mathematisches Distanzmaß wählen kann, wird das erfindungsgemäße Verfahren flexibler einsetzbar und anpassbar. Insbesondere kann ein Benutzer dabei seine Erfahrungen einfließen lassen, um ein geeignetes mathematisches Distanzmaß auszuwählen, um eine Bestimmung der Stabilität eines Stoffgemischs mit der dem erfindungsgemäßen Verfahren durchzuführen.

Insbesondere kann vorgesehen sein, dass der Benutzer für die Quantifizierung der Veränderung des jeweiligen Stoffgemischs auch iterativ nacheinander mehrere mathematische Distanzmaße wählt, wobei die Quantifizierung auf Basis jedes gewählten mathematischen Distanzmaßes erfolgt. So wird es einem Benutzer ermöglicht, die Ergebnisse verschiedener wählbarer mathematischer Distanzmaße miteinander zu vergleichen. Somit wird das Verfahren zur Bestimmung der Stabilität eines Stoffgemischs besonders flexibel und vielseitig. Durch das Vorsehen dieser Möglichkeit kann ferner eine unbeabsichtigte Auswahl eines mathematischen Distanzmaßes zur Quantifizierung einer Veränderung jeweiliger Stoffgemische geändert werden.

Vorzugsweise umfasst das Verfahren ferner einen Datenvorverarbeitungsschritt, Dieser kann ein Durchführen einer Streulichtkorrektur des zumindest einen Messdatensatzes umfassen, insbesondere wenn dieser Nahinfrarot-Messdaten umfasst. Ferner kann der Datenvorverarbeitungsschritt ein Durchführen einer Zentrierung, Normierung und/oder Skalierung des zumindest einen Messdatensatzes umfassen. Des Weiteren kann der Datenvorverarbeitungsschritt auch ein Durchführen einer Hauptkomponentenanalyse umfassen.

Als Datenvorverarbeitung wird vorliegend eine mathematische Verarbeitung von Rohdaten, die zum Ziel hat, die eigentliche Auswertung vorzubereiten, verstanden. Diese kann beispielsweise eine Streulichtkorrektur, eine Erhöhung des Signal-Rausch-Abstands oder eine bloße Umformatierung erfasster Messdatensätze bzw. Rohdaten beinhalten, so dass bei einer nachgelagerten Auswertung eingespeiste Daten vom Algorithmus korrekt verarbeitet werden können. Eine geeignete Datenvorverarbeitung kann insbesondere zur Erhöhung der Qualität der Ergebnisse des erfindungsgemäßen Verfahrens eingesetzt werden. Die Datenvorverarbeitung erfolgt vorzugsweise nach dem Datenerfassungsschritt des erfindungsgemäßen Verfahrens.

Durch entsprechende Datenvorverarbeitung, die insbesondere eine Reduktion der Datenmenge umfassen kann, sollen mögliche Parameterinteraktionen innerhalb eines komplexen Gesamtdatensatzes automatisch miterfasst und berücksichtigt werden. Mit anderen Worten: Die Genauigkeit des erfindungsgemäßen Verfahrens bzw. die Auswertungsgüte kann erhöht werden, indem insbesondere vor der eigentlichen Datenauswertung eine Streulichtkorrektur (beispielsweise bei Nahinfrarotspektroskopie-Daten) durchgeführt wird.

Auch eine Normierung oder Skalierung der Daten, sowie eine vorgeschaltete Principal Component Analysis kann unerwünschtes Rauschen von Informationen eines Datensatzes abtrennen und so die Aussagegüte, -validität und Robustheit weiter steigern. Als Rauschen wird eine Störgröße mit breitem, unspezifischem Frequenzspektrum, die gegebenenfalls gewünschte, informationstragende Signale überlagern oder maskieren können, verstanden. Ein Beispiel für Rauschen ist unerwünschtes Streulicht, das neben gewünschtem Anregungslicht zufällig auf den Detektor eines Nahinfrarotspektroskopie-Geräts fällt und zusätzlich gemessen wird. Die Anwendung spezieller mathematischer Techniken kann helfen, Rauschen von verwertbarer Information abzutrennen und so den Signal-Rausch-Abstand zu vergrößern und eine Auswertung robuster / vertrauenswürdiger zu machen.

Es kann ferner vorgesehen sein, dass der Datenausgabeschritt umfasst: Darstellen der Veränderung des jeweiligen Stoffgemischs als Box-Plot; und/oder Darstellen von Mittelwerten, Medianen, 0,25-/0,75-Quantilen, Hervorheben möglicher Ausreißer-Kandidaten; und/oder Durchführen zumindest eines statistischen Tests, insbesondere t-Test, Wilcoxon-Rang-Summen-Test, One-Way-ANOVA und/oder Kruskall-Wallis-Test.

Durch diese Ausgestaltung des Datenausgabeschritts wird die Untersuchung sehr großer Messdatensätze, insbesondere bei einer Mehrzahl von Proben, mit höherer Aussagekraft bei gleichzeitig geringerem Zeitaufwand ermöglicht. Ferner wird in vorteilhafter Weise eine intuitive Auswertung bereitgestellt. Das bedeutet, dass diese Technologie von Anwendern ohne tiefere mathematische Kenntnisse nutzbar ist und auch keine individuelle Programmierung für jede Untersuchung erforderlich ist. Ein Anwender kann, beispielsweise bei einem Datenausgabeschritt, der die Veränderung des untersuchten Stoffgemischs als Box-Plot ausgibt, auf einen Blick und auf einfache intuitive Weise Erkenntnisse gewinnen. Werden Mittelwerte, Mediane, 0,25-/0,75-Quantile und Ausreißer hervorgehoben, wird die Anwenderfreundlichkeit weiter erhöht. Von einem Anwender erhobene Messdatensätze können beispielsweise direkt von einem Messgerät oder nach einem Datenvorverarbeitungsschritt, wie oben beschrieben, in das Verfahren eingeschleust werden und es wird sofort eine leicht interpretierbare Auswertung erzeugt.

Es kann ferner vorgesehen sein, dass das Bestimmen des Startwerts des jeweiligen Stoffgemischs auf Basis von Metadaten des jeweiligen Messdatensatzes basiert.

Durch die Verwendung von Metadaten bei der Bestimmung des Startwerts kann in vorteilhafter Weise Untersuchungszeit bzw. Verarbeitungszeit eingespart werden. Der Startwert wird in kurzer Zeit auf Basis von Metadaten ermittelt.

Vorzugsweise ist vorgesehen, dass der Datenauswertungsschritt ferner umfasst: Durchführen einer zusätzlichen Hauptkomponentenanalyse für den Messdatensatz, welche nicht in das Quantifizieren der Veränderung mittels des mathematischen Distanzmaßes eingeht, wobei im Datenausgabeschritt das Ergebnis der zusätzlichen Hauptkomponentenanalyse dargestellt wird.

Durch die zusätzliche Hauptkomponentenanalyse, die insbesondere eine qualitative Analyse ist, die nicht in die Distanzberechnung eingeht, kann die Auswertung ausgegebener Ergebnisse nach dem Datenausgabeschritt durch den Anwender unterstützt werden. Durch den Datenausgabeschritt erhält der Anwender somit einerseits eine quantitative Analyse, die insbesondere das Distanzmaß umfasst, und andererseits eine qualitative Analyse, die weitere Rückschlüsse auf einen Blick und auf intuitive Weise ermöglicht.

Es kann insbesondere vorgesehen sein, dass das Stoffgemisch feste und/oder flüssige und/oder gasförmige Stoffgemische umfasst.

Es kann ferner vorgesehen sein, dass das Stoffgemisch biologische, chemische, pflanzliche, tierische, humane Stoffe oder Stoffgemische, pharmazeutische Zusammensetzungen, pflanzliche Arzneimittel, chemische und/oder biologische Arzneimittel, Zellen, Zelltherapeutika (beispielweise Gentherapeutika, z.B. CAR T-Zellen (Chimeric Antigen Receptor T-Zellen), NK-Zellen (Natural Killer-Zellen) somatische Zelltherapeutika, biotechnologisch bearbeitete Gewebeprodukte/tissue engineered products, Gewebe, Stammzellen, Stammzellprodukte bzw. -zubereitungen, z.B. CD34+-Zellen, CD19+-Zellen, CD20+-Zellen, HEK295-Zellen, TCR alpha/beta-Zellen, TCR gamma/delta-Zellen, CD3+-, CD4+-, CD8+- CD133+-Zellen), Blut, Blutprodukte, Organe, Arzneitees, Extrakte, insbesondere Eisenkrautextrakt, Tropfen, Tabletten, Dragees, Kapseln, Pulver, Granulate, Lösungen, Suspensionen, Säfte, Nahrungsmittel, insbesondere Fleisch oder Hackfleisch, Fruchtsaft, insbesondere Orangensaft, Nahrungsergänzungsmittel, Kosmetika, Emulsionen, Salben und/oder Cremes umfasst, sowie Verpackungen, Packmittel, Folien, insbesondere Polyethylen, Polyvinylchlorid, usw.

Das Verfahren zur Bestimmung der Stabilität eines Stoffgemischs ist sehr vielseitig einsetzbar. So können vorteilhaft Stoffgemische in jedem Aggregatzustand untersucht werden.

Das erfindungsgemäße Computerprogramm umfasst Befehle, die bei der Ausführung des Programms durch einen Computer diesen veranlassen, das Verfahren wie oben beschrieben, auszuführen.

Die erfindungsgemäße Vorrichtung kann insbesondere ein Messinstrument oder ein Server-Computer sein und umfasst Mittel zur Ausführung des Verfahrens wie oben beschrieben. Als erfindungsgemäße Vorrichtung ist ferner auch ein mobiles elektronisches Gerät, wie beispielsweise ein Smartphone, eine speicherprogrammierbare Steuerung oder ein sog. "Edge-Device", denkbar. Zusätzlich kann das Verfahren auch als Cloudlösung im Rahmen von "Software-as-a-Service" oder generell als eine "serverless"-Anwendung bereitgestellt werden.

Die in Bezug auf das erfindungsgemäße Verfahren beschriebenen technischen Vorteile und Ausgestaltungen gelten gleichermaßen für das erfindungsgemäße Computerprogramm sowie für die erfindungsgemäße Vorrichtung.

## KURZE BESCHREIBUNG DER ZEICHNUNGEN

Bevorzugte Ausführungsformen der vorliegenden Offenbarung werden nachfolgend mit Bezugnahme auf die folgenden Zeichnungen beschrieben:

Fig. 1     zeigt schematisch eine grobe Übersicht über mögliche Schritte des Verfahrens gemäß Ausführungsformen der Erfindung.

Fig. 2     zeigt schematisch eine Übersicht über mögliche Schritte des Verfahrens gemäß Ausführungsformen der Erfindung.

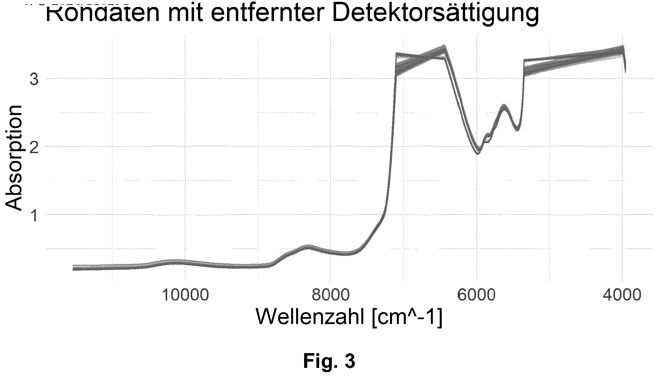

Fig. 3     zeigt ein Spektrenoverlay gemessener Messdatensätze eines Nahinfrarotspektroskopie-Geräts mit entfernter Detektorsättigung.

Fig. 4     zeigt ein Spektrenoverlay gemessener Messdatensätze eines Nahinfrarotspektroskopie-Geräts ohne Datenvorverarbeitung (oben) und mit beispielhafter Datenvorverarbeitung (Streulichtkorrektur) (unten).

Fig. 5     zeigt den Boxplot eines Vergleichs der ermittelten, euklidischen Distanzen, berechnet auf Basis von NIR-Spektren von Orangensaft, der über mehrere Tage bei Raumtemperatur (="RT"), bzw. 40°C (="40C") gelagert wurde ("SW"=Startwert).

Fig. 6     zeigt den Mittelwert +/- Standardfehler der ermittelten, euklidischen Distanzen, berechnet auf Basis von NIR-Spektren von Hackfleischproben, die über mehrere Tage bei Raumtemperatur (="RT"), bzw. 40°C (="40C") gelagert wurden ("SW"=Startwert).

Fig. 7     zeigt die photographische Veränderung der Hackfleischproben über den beobachteten Zeitraum bei 40 °C und Raumtemperatur (RT). Es ist eine deutliche Veränderung der Probe bei 40 °C zu erkennen (1). Bei RT bleibt die Marmorierung des Hackfleischs deutlich erhalten (2 und 3). Mit dem erfindungsgemäßen Verfahren lässt sich dieser qualitative Verfall jedoch wesentlich präziser quantifizieren und verfolgen.

Fig. 8     zeigt den Boxplot eines Vergleichs der ermittelten, euklidischen Distanzen, berechnet auf Basis von NIR-Spektren aus einem Extraktionsversuch von Eisenkraut, der über einen definierten Zeitraum von 180 Minuten durchgeführt wurde.

Fig. 9     zeigt eine nähere kinetische Auswertung der Daten aus Fig. 8, bei der insbesondere die Geschwindigkeitskonstanten ($Distanz_{max}$ und k) des Versuchs ermittelt wurden, sowie daraus abgeleitet die geschätzten notwendigen Zeiten für eine 50-, 90- und 95-prozentige Extraktion.

Fig. 10     zeigt eine nähere kinetische Auswertung von Daten aus einem Zellkulturversuch, hier eine unbehandelte HEK295-Zellkultur ohne zusätzliches Stress-Agens (=Kontrolle). Ermittelt wurden die Zeiten bis zur 50%-($K_m$), 90%- und 95%-Erreichung eines Plateauwerts.

Fig. 11     zeigt die nähere kinetische Auswertung von Daten aus einem Zellkulturversuch unter Einfluss einer 3% Ethanol-Lösung, die als Stress-Agens hinzugefügt wurde. Ermittelt wurden die Zeiten bis zur 50%- ($K_m$), 90%- und 95%- Erreichung eines Plateauwerts. Die unterschiedlichen Viabilitäten und Kinetiken (siehe auch Fig. 12) lassen sich dabei sehr gut mit dem Vorversuch (Ausführungsbeispiel 4), bei dem noch wesentlich mehr Konzentrationen getestet wurden, korrelieren.

Fig. 12     zeigt die nähere kinetische Auswertung von Daten aus einem Zellkulturversuch unter Einfluss einer 6% Ethanol-Lösung, die als Stress-Agens hinzugefügt wurde. Ermittelt wurden die Zeiten bis zur 50%- ($K_m$), 90%- und 95%- Erreichung eines Plateauwerts.

Fig. 13     zeigt die Zusammenfassung der ermittelten Plateauwerte aus dem Zellkulturversuch. Der ermittelte Distanzwert für den Ansatz mit 6% Ethanol ist am größten, was auf einen starken Einfluss dieser hohen Konzentration auf die Zellkultur schließen lässt. Dieser Messansatz ist also am "instabilsten". Der Wert für den 3%- und Kontroll-Ansatz ist entsprechend geringer. Diese Ergebnisse werden auch durch den zuvor durchgeführten Zellviabilitätsassay widergespiegelt.

Fig. 14     zeigt die Korrelation zwischen Zellviabilität und eingesetzter Ethanolkonzentration (in %) im Vorversuch (A), beobachteter maximaler euklidischer Distanz und eingesetzter Ethanolkonzentration (in %) im Hauptversuch (B), sowie die Korrelation von Zellviabilität und beobachteter maximaler euklidischer Distanz (C), die beide analytischen Techniken in Relation setzt. Für jede Untergraphik (A), (B) und (C) wurde eine lineare Trendlinie hinzugefügt, die jeweils ein $R^2$ von 0,955, 0,999, bzw. 0,999 aufweist.

Fig. 15     Boxplot der euklidischen Distanzen einer NIR-Messung einer Arzneimitteltee-Zubereitung bei verschiedenen Lagerbedingungen (Kühlschrank (KS)/ Raumtemperatur (RT) / 40°C Klimaschrank).

Fig. 16     Vergleich von Stabilitätsmessungen einer Arzneimitteltee-Zubereitung. Gemessen wurde mittels einer validierten Referenzmethode (photometrischer Assay im UV/VIS-Bereich, in Legende bezeichnet als GPP), sowie mittels NIR unter Verwendung der erfindungsgemäßen neuen Auswertungsmethode.

Fig. 17     Beispielhafte graphische Darstellung des HPLC/MS-Rohdatensatzes zum 20%-igem ethanolischen Auszug einer Arzneidrogenmischung aus Thymian, Rosmarin und Kamille. Diese Rohdaten wurden direkt über das neuartige Auswerteverfahren verarbeitet.

Fig. 18 Boxplotdarstellung der berechneten euklidischen Distanzen zu dem in Fig. 17 dargestellten HPLC/MS-Datensatz zum 20%-igen ethanolischen Auszug. Die euklidischen Distanzen wurden gegen die Wochenwerte (Beobachtungszeitraum 24 Wochen) bzw. ganz rechts nach Bestrahlung mit Licht (SunT) aufgetragen. Erläuterung Legende: KII = Klimazone II; AC = Klimazone AC; B = Braunglasflasche; W = Weißglasflasche.

Fig. 19 Fit der ermittelten Kinetikfunktion für die euklidischen Distanzen berechnet aus den HPLC/MS-Rohdaten für den 20%-igen ethanolischen Auszug aus Fig. 17. Erläuterung Legende: KII = Klimazone II; AC = Klimazone AC.

Fig. 20 Stabilitätsverlauf des 20%-igen Auszugs einer Arzneidrogenmischung aus Thymian, Rosmarin und Kamille über eine stellvertretende, einzelne, selektierte Markerverbindung (m/z 329,17; Retentionszeit = 8,16 min) bei unterschiedlichen klimatischen Bedingungen.

Fig. 21 Boxplotdarstellung der euklidischen Distanzen einer Arzneidrogenpulvermischung aus Rosmarin, Thymian und Kamille vor (Zeitpunkt 0) und nach Bestrahlung mit Licht über 20 Stunden (Zeitpunkt 20). Erläuterung Legende: P1 = Pulvermischung in Papiertüte verpackt; P2 = Pulvermischung offen gelagert.

Fig. 22 Säulendiagramm zu Referenzdaten mittels nasschemisch-photometrischer Erfassung der Gesamtpolyphenole in Anlehnung an die Monographie 2.8.14 des europäischen Arzneibuchs. Es wurde eine Arzneidrogenpulvermischung über 20 Stunden mit Licht zum einen in einer Papiertüte verpackt (P1) und zum anderen offen gelagert (P2) bestrahlt. Die Wiederfindung ist zu interpretieren als prozentuale Übereinstimmung der Absorption nach der Bestrahlung zu dem Ausgangswert der unbelasteten Probe.

Fig. 23 Fit der ermittelten Kinetikfunktion für die auf latenten Variablen berechneten euklidischen Distanzen auf Basis der NIR-Spektren (SNV-korrigiert) für eine Arzneidrogenpulvermischung über einen Beobachtungszeitraum von 24 Wochen bei unterschiedlichen Klimazonen. Erläuterung Legende: KII = Klimazone II; AC = Klimazone AC.

Fig. 24 Stabilitätsverlauf einer Arzneidrogen-Pulvermischung über eine stellvertretende, einzelne, selektierten Markerverbindung (m/z 329,17; Retentionszeit = 8,16 min) bei unterschiedlichen klimatischen Bedingungen.

Fig. 25 Optische Veränderung einer Arzneidrogenpulvermischung nach Einlagerung bei unterschiedlichen Klimazonen nach 24 Wochen. Die Probe der bei Klimazone AC gelagerten Mischung ist deutlich dunkler verfärbt.

Fig. 26 Boxplotdarstellung der berechneten euklidischen Distanzen aus einem NIR-Datensatz (SNV-korrigiert) Weißdorn Filmtabletten. Die euklidischen Distanzen wurden gegen die Wochenwerte (Beobachtungszeitraum 24 Wochen) aufgetragen. Erläuterung Legende: FT = Filmtabletten in Polyethylen-Beutel; PP = Filmtabletten in Blister und Faltschachtel.

Fig. 27 Stabilitätsverlauf von Weißdorn-Filmtabletten über ein stellvertretendes dimeres Procyanidin (m/z 577,14; Retentionszeit = 2,69 min) bei unterschiedlichen klimatischen Bedingungen und unterschiedlichen Packmitteln. Erläuterung Legende: FT = Filmtabletten in Polyethylen-Beutel; PP = Filmtabletten in Blister und Faltschachtel.

## BESCHREIBUNG BEVORZUGTER AUSFÜHRUNGSBEISPIELE

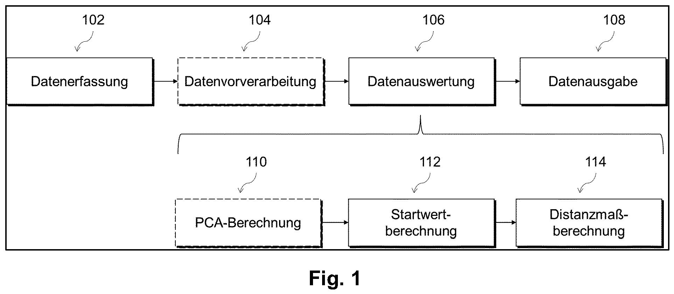

Wie in Fig. 1 dargestellt, erfolgt zunächst eine Datenerfassung 102. Dabei wird zumindest ein Messdatensatz empfangen, der durch eine Messung mittels eines oder mehrerer Messgeräte generiert werden kann. Jeder Messdatensatz umfasst ein chemisches, insbesondere phytochemisches Profil des zu untersuchenden Stoffgemischs. Der Messdatensatz bzw. die mehreren Messdatensätze können insbesondere mittels Nahinfrarotspektroskopie erhaltene Daten umfassen. Durch eine Nahinfrarotspektroskopie (NIR) können Proben von Stoffen oder Stoffgemischen ohne komplizierte Probenaufarbeitung gemessen werden, um Messdatensätze zu erzeugen. Diese Technologie erfasst Kombinations- und Obertonschwingungen aller Moleküle einer Probe und ist daher in der Lage, diese ganzheitlich zu erfassen. Der Vorteil von Datensätzen, die beispielsweise mittels einer Nahinfrarotspektroskopie (NIR) gewonnen werden besteht darin, dass diese Datensätze - verglichen mit Datensätzen, die aus anderen analytischen Techniken gewonnen werden - sehr viel mehr Informationen in einem Datum einer Beobachtung enthalten, denn klassische analytische Techniken beobachten nur wenige isolierte Signale gleichzeitig. Die Nahinfrarotspektroskopie ermöglicht mit anderen Worten eine breitere Auswertung einer Vielzahl von Signalen.

Alternativ oder zusätzlich kann der Messdatensatz bzw. die mehreren Messdatensätze Daten umfassen, die mittels UV/VIS-Spektroskopie, Raman-Spektroskopie, (U)HPLC-Fingerprints (insbesondere aus einer Kopplung mit unterschiedlichen Detektoren, zum Beispiel DAD, MS, ELSD), GC-Fingerprints (insbesondere aus einer Kopplung mit diversen Detektoren, zum Beispiel FID, MS) oder ganz generell Peaktables aus allen denkbaren chromatographischen Verfahren, erfasst wurden. Das Verfahren kann mit anderen Worten auf eine Vielzahl von Messtechniken angewendet werden, die ein oder mehrere X-Werte (beispielsweise Wellenlängen, m/z-Verhältnisse, Retentionszeiten, Messpunkte) ein oder mehreren Y-Werte (beispielsweise Intensitäten, Absorptionen, Spannungen, Messwerte) zuordnen. Das Verfahren ist somit besonders vielseitig und flexibel einsetzbar.

In einem weiteren, optionalen Schritt, kann eine Datenvorverarbeitung 104 erfolgen. Diese kann insbesondere umfassen: Durchführen einer Streulichtkorrektur des zumindest einen Messdatensatzes, insbesondere wenn dieser Nahinfrarot-Messdaten umfasst; Durchführen einer Zentrierung, Normierung und/oder Skalierung des zumindest einen Messdatensatzes; und/oder Durchführen einer Hauptkomponentenanalyse. Eine geeignete Datenvorverarbeitung kann insbesondere zur Erhöhung der Qualität der Ergebnisse des erfindungsgemäßen Verfahrens eingesetzt werden. Durch die Datenvorverarbeitung, die insbesondere eine Reduktion der Datenmenge umfassen kann, sollen mögliche Parameterinteraktionen innerhalb eines komplexen Gesamtdatensatzes automatisch miterfasst und berücksichtigt werden. Mit anderen Worten: Die Genauigkeit des erfindungsgemäßen Verfahrens bzw. die Auswertungsgüte kann erhöht werden, indem insbesondere vor der eigentlichen Datenauswertung eine Streulichtkorrektur (beispielsweise bei Nahinfrarotspektroskopie-Daten) durchgeführt wird.

In einem weiteren Schritt erfolgt eine Datenauswertung 106 für jeden Messdatensatz. Dabei erfolgt eine Startwertberechnung 112 und eine Distanzmaßberechnung 114, jeweils für jeden Messdatensatz. Bei der Startwertberechnung 112 wird auf Basis der gemessenen Messdaten ein Startwert bestimmt, von dem ausgehend eine Veränderung des zu untersuchenden Stoffgemischs beobachtet wird und im Rahmen der Distanzmaßberechnung 114 quantifiziert wird. Die Distanzmaßberechnung 114 kann insbesondere auf der Gesamtheit der Inhaltsstoffe des zu untersuchenden Stoffgemischs erfolgen, um die Robustheit und Validität des Ergebnisses zu steigern.

Das Distanzmaß bei der Distanzmaßberechnung 114 kann insbesondere gewählt sein aus der folgenden Gruppe: euklidische Distanz, Mahalanobis-Distanz, Manhattan-Distanz, Pearson-Distanz und/oder Gower-Distanz. Durch die Auswahl eines mathematischen Distanzmaßes aus der eben genannten Gruppe wird gewährleistet, dass die Veränderung eines zu untersuchenden Stoffs oder Stoffgemischs in einem zeitlichen Verlauf quantifiziert wird, wobei die Komplexität der zugrundeliegenden Datensätze ganzheitlich verarbeitet werden kann. Die Wahl einer geeigneten mathematische Strategie den Grad der Veränderung einer Probe in einem zeitlichen Verlauf zu quantifizieren ermöglicht somit eine effiziente Quantifizierung der Veränderung eines jeweiligen Stoffs oder Stoffgemischs. Es wird insbesondere ein sehr guter vergleichender Überblick über einen Datensatz einer Stabilitätsuntersuchung gewonnen, ohne eine Vielzahl separater Parameter einzeln prüfen zu müssen.

Besonders vorteilhaft ist vorgesehen, dass das mathematische Distanzmaß durch einen Benutzer auswählbar ist.

Durch die Möglichkeit, dass ein Benutzer ein mathematisches Distanzmaß wählen kann, wird das erfindungsgemäße Verfahren flexibler einsetzbar und anpassbar. Insbesondere kann ein Benutzer dabei seine Erfahrungen einfließen lassen, um ein geeignetes mathematisches Distanzmaß auszuwählen, um eine Bestimmung der Stabilität eines Stoffs oder Stoffgemischs mit der dem erfindungsgemäßen Verfahren durchzuführen.

Insbesondere kann vorgesehen sein, dass der Benutzer für die Quantifizierung der Veränderung des jeweiligen Stoffs oder Stoffgemischs mehrere mathematische Distanzmaße wählt, wobei die Quantifizierung auf Basis jedes gewählten mathematischen Distanzmaßes erfolgt. So wird es einem Benutzer ermöglicht, die Ergebnisse verschiedener wählbarer mathematischer Distanzmaße miteinander zu vergleichen.

Alternativ oder zusätzlich kann vorgesehen sein, dass der Benutzer nacheinander verschiedene mathematische Distanzmaße wählt, um die Veränderung der jeweiligen Stoffe oder Stoffgemische zu quantifizieren. Somit wird das Verfahren zur Bestimmung der Stabilität eines Stoffs oder Stoffgemischs besonders flexibel und vielseitig. Durch das Vorsehen dieser Möglichkeit kann ferner eine unbeabsichtigte Auswahl eines mathematischen Distanzmaßes zur Quantifizierung einer Veränderung jeweiliger Stoffe oder Stoffgemische geändert werden.

Optional kann im Rahmen der Datenauswertung 106 eine PCA-Berechnung 110 erfolgen, die der Startwertberechnung 112 und der Distanzmaßberechnung 114 vorgelagert ist. Die Abkürzung PCA steht für Principal Component Analysis (deutscher Begriff: Hauptkomponentenanalyse). Die Datenauswertung 106 kann somit sowohl mit den Originaldaten durchgeführt werden als auch nach einer Datenskalierung und/oder Datenreduktion auf latente Variablen, beispielsweise durch die PCA-Berechnung 110.

In einem letzten in Fig. 1 dargestellten Schritt erfolgt die Datenausgabe 108. Dabei wird für jeden Messdatensatz die Veränderung des jeweiligen Stoffgemischs grafisch dargestellt. Die Datenausgabe 108 kann insbesondere umfassen: Darstellen der Veränderung des jeweiligen Stoffgemischs als Box-Plot; und/oder Darstellen von Mittelwerten, Medianen, 0,25-/0,75-Quantilen, Hervorheben möglicher Ausreißer-Kandidaten; und/oder Durchführen zumindest eines statistischen Tests, insbesondere t-Test, Wilcoxon-Rang-Summen-Test, One-Way-ANOVA und/oder Kruskall-Wallis-Test. Durch diese Ausgestaltung der Datenausgabe 108 wird die die Untersuchung sehr großer Messdatensätze, insbesondere bei einer Mehrzahl von Proben, mit höherer Aussagekraft bei gleichzeitig geringerem Zeitaufwand ermöglicht. Ferner wird in vorteilhafter Weise eine intuitive Auswertung bereitgestellt. Das bedeutet, dass diese Technologie von Anwendern ohne tiefere mathematische Kenntnisse nutzbar ist und auch keine individuelle Programmierung für jede Untersuchung erforderlich ist. Ein Anwender kann, beispielsweise bei einem Datenausgabeschritt, der die Veränderung des untersuchten Stoffgemischs als Box-Plot ausgibt, auf einen Blick und auf einfache intuitive Weise Erkenntnisse gewinnen. Werden Mittelwerte, Mediane, 0,25-/0,75-Quantile und Ausreißer hervorgehoben, wird die Anwenderfreundlichkeit weiter erhöht. Von einem Anwender erhobene Messdatensätze können beispielsweise direkt von einem Messgerät oder nach einem Datenvorverarbeitungsschritt, wie oben beschrieben, in das Verfahren eingeschleust werden und es wird sofort eine leicht interpretierbare Auswertung erzeugt.

Fig. 2 zeigt schematisch eine Übersicht über mögliche Schritte des Verfahrens gemäß Ausführungsformen der Erfindung. Die vorliegende Erfindung wird nachfolgend anhand der Fig. 2 erläutert.

In einem Schritt 202 werden Rohdaten aus einem oder mehreren Messgeräten zusammengestellt. Es können, wie oben beschrieben, zur Erfassung der Rohdaten (Datenerfassungsschritt) verschiedene Messungen desselben Stoffgemischs oder verschiedener Proben eines Stoffgemisch durchgeführt werden.

In einem Schritt 204 ist zu entscheiden, ob eine Vorverarbeitung gewünscht ist oder nicht. Ist keine Vorverarbeitung gewünscht, wird in einem nächsten Schritt 211 das Distanzmaß gewählt. Für die Wahl des Distanzmaßes kann aus den oben genannten Distanzmaßen gewählt werden. Nachdem die Wahl des Distanzmaßes erfolgt ist, erfolgen - je nach Ausgestaltung des Verfahrens - eine quantitative und/oder eine qualitative Auswertung. Im Rahmen der quantitativen Auswertung werden zunächst in einem Schritt 212 für jeden Messdatensatz Startwerte bzw. Nullwerte identifiziert. In einem weiteren Schritt 214 wird, beispielsweise über die Zeit gesehen, die Veränderung eines zu untersuchenden Stoffgemischs anhand des gewählten Distanzmaßes ermittelt. Das Ergebnis wird in einem Schritt 216 beispielsweise in einem Boxplot gruppiert und dargestellt. Zusätzlich können im Rahmen der quantitativen Auswertung begleitende statistische Tests in einem Schritt 217 durchgeführt werden. Im Rahmen einer möglichen qualitativen Auswertung 316, beispielsweise durch eine zusätzliche Hauptkomponentenanalyse, kann ein Anwender eine neben der quantitativen Auswertung nützliche Aussage erhalten. In einem Schritt 400 werden die Resultate bzw. Ergebnisse grafisch dargestellt und ausgegeben.

Wird in Schritt 204 entschieden, dass eine Datenvorverarbeitung gewünscht ist, wird zunächst in einem Schritt 2041 entschieden, ob ein Zuschnitt der Messdatensätze gewünscht ist. Ist ein Zuschnitt der Messdatensätze gewünscht, so wird dieser in einem Schritt 2042 durchgeführt. Anschließend ist in einem Schritt 2043 zu entscheiden, ob eine Sättigungskorrektur gewünscht ist, wobei wenn eine Sättigungskorrektur gewünscht ist, diese in einem Schritt 2044 durchgeführt wird. Ferner wird in einem Schritt 2045 entschieden, ob eine Streulichtkorrektur gewünscht ist. Wird in Schritt 2045 eine Streulichtkorrektur gewünscht, so wird diese in einem Schritt 2046 durchgeführt. Anschließend wird in einem Schritt 2047 entschieden, ob eine Ableitung mit einer Glättung der Messdatensätze gewünscht ist, wobei wenn eine solche gewünscht ist, diese in einem Schritt 2048 durchgeführt wird. Ferner wird in einem Schritt 2049 entschieden, ob eine Berechnung auf latenten Variablen gewünscht ist und es wird in einem Schritt 2050 eine Skalierung gewählt. Die Skalierung kann beispielsweise gewählt sein aus "keine", "UV" (="univarianz", nicht zu verwechseln mit "ultaviolett")" oder "Pareto". Es versteht sich, dass bei der optionalen Vorverarbeitung auch nur eine Auswahl der oben geschriebenen Maßnahmen durchgeführt werden kann.

In einem Schritt 210 kann anschließend eine PCA-Berechnung erfolgen. Anschließend wird, wie oben beschrieben, mit der Wahl des Distanzmaßes fortgefahren.

IMPLEMENTIERUNGSBEISPIEL

Die nachfolgend erläuterten Versuche wurden mittels einer Referenzimplementierung in der Programmiersprache R durchgeführt. Die Referenzimplementierung beinhaltet die folgenden Schritte:

- Berechnung der Distanzen der einzelnen Beobachtungen / Proben über geeignete Distanzmaße (beispielsweise Euklid, Mahalanobis, Manhattan, Pearson, Gower, o. ä.) zu ihrem Startwert ("Null-Monatswert"). Diese Distanzberechnung kann sowohl auf Rohdaten als auch auf den latenten Variablen einer Vorverarbeitung (beispielsweise PCA, Datenskalierung, o. ä.) erfolgen. Aus den berechneten Distanzmaßen besteht auch die Möglichkeit, die Kinetik des Abbaus eines Produkts zu bestimmen. Auf diese Weise können beispielsweise auch Halbwertszeiten zur Identifizierung der stabilsten Variante(n) herangezogen werden.

- Einfach zu interpretierende Darstellung der berechneten Distanzen in Box-Plots inkl. automatisches Durchführen der deskriptiven Statistik (Berechnung und Darstellung von Mittelwerten, Medianen, Darstellung der 0,25-/0,75-Quantile, Einzeichnung möglicher Ausreißer-Kandidaten) und festlegen / durchführen des am besten geeigneten statistischen Test (t-Test / Wilcoxon-Rang-Summen-test / One-Way-ANOVA / Kruskall-Wallis-Test) zum Vergleich der Mittelwerte.

- Möglichkeit zur Verfolgung der Änderungen der Proben im zeitlichen Verlauf inklusive Abschätzung der statistischen Signifikanz (beispielsweise ob nach n Monaten noch weitere Degradation in den Proben auftritt oder ein Plateau erreicht wurde)

- Überführen der spektralen Daten unter Verwendung einer Hauptkomponentenanalyse (PCA) in latente Variablen, um über Score-Plots zusätzlich unterschiedliche Arten von Abbau- oder Umlagerungsmechanismen abschätzen zu können. Hier können zwei Fälle unterschieden werden:

A) Es kann aus den Rohdaten eine PCA errechnet werden, deren Score-Werte dann in die Distanzberechnung und späteren Boxplot-Darstellung & Statistik eingehen. Dann werden diese Scores auch graphisch angezeigt. B) Die eigentliche Distanzberechnung (und Boxplot-Darstellung, sowie der zugehörigen Statistik) kann auch ohne vorgeschaltete PCA erfolgen - diese wird dann trotzdem berechnet und angezeigt, geht aber eben nicht in die eigentlich kritische Distanzberechnung ein.

- Die erstellte App ermöglicht eine intuitive Auswertung ohne Programmier- oder tiefere mathematische Kenntnisse für die Endanwender.

Das korrespondierende Berechnungsverfahren der Implementierung in einer Ausführungsform der Erfindung wird nachfolgend beschrieben:

Ausgangspunkt ist in diesem Ausführungsbeispiel eine vom Nutzer vorbereitete Rohdatentabelle, die die zu untersuchenden Proben und Metainformationen (beispielsweise in welcher Klimazone oder in welchem Monat die Probe gemessen wurde) enthält. Jede Zeile ist dabei eine Probe, jede Spalte ist eine "Eigenschaft", beispielsweise Intensität bei einer gemessenen Wellenlänge oder Zahlenwert der Bruchfestigkeit, Konzentration einer enthaltenen Substanz oder andere erhobene Parameter. Die Anzahl der Spalten kann je nach Messverfahren und Verfügbarkeit / Relevanz zusätzlicher Metainformationen variabel sein. Der Ausschnitt einer beispielhaften Rohdatentabelle ist hier gezeigt:

*Tabelle 1: Exemplarischer Ausschnitt des Rohdatensatzes zu dem Anwendungsbeispiel 1. Dieser umfasst sowohl die Metainformationen als auch die NIR-Spektren zu unterschiedlichen Zeitpunkten bei unterschiedlichen klimatischen Bedingungen bzgl. einer Stabilitätsstudie zu Orangensaft.*

| Metainformation | | | Wellenzahl [cm$^{-1}$] | | |
|---|---|---|---|---|---|
| **Produkt** | **Zeit (in Tagen)** | **Klima** | **11544** | **...weitere Wellenzahlen ...** | **3952** |
| Orangensaft | 0 | SW | 2,4833 | ... weitere Intensitätswerte... | 4,61342 |
| Orangensaft | 0 | SW | 2,51817 | ... weitere Intensitätswerte... | 4,38362 |
| Orangensaft | 1 | 40 °C | 2,56125 | ... weitere Intensitätswerte... | 4,68658 |
| Orangensaft | 1 | 40 °C | 2,576 | ... weitere Intensitätswerte... | 4,77443 |
| Orangensaft | 1 | RT | 2,52725 | ... weitere Intensitätswerte... | 4,41684 |
| Orangensaft | 1 | RT | 2,5428 | ... weitere Intensitätswerte... | 4,78359 |
| Orangensaft | 2 | 40 °C | 2,62842 | ... weitere Intensitätswerte... | 4,44054 |
| Orangensaft | 2 | 40 °C | 2,54003 | ... weitere Intensitätswerte... | 4,39748 |
| Orangensaft | 2 | RT | 2,61846 | ... weitere Intensitätswerte... | 4,66339 |
| Orangensaft | 2 | RT | 2,57705 | ... weitere Intensitätswerte... | 4,59864 |
| Orangensaft | 3 | 40 °C | 2,58429 | ... weitere Intensitätswerte... | 4,44866 |
| Orangensaft | 3 | 40 °C | 2,57807 | ... weitere Intensitätswerte... | 4,70549 |
| Orangensaft | 3 | RT | 2,59324 | ... weitere Intensitätswerte... | 4,42383 |
| Orangensaft | 3 | RT | 2,59459 | ... weitere Intensitätswerte... | 4,8207 |
| Orangensaft | 4 | 40 °C | 2,61964 | ... weitere Intensitätswerte... | 4,77069 |
| Orangensaft | 4 | 40 °C | 2,64142 | ... weitere Intensitätswerte... | 4,46515 |

**EP 4 515 551 B1**

(fortgesetzt)

| Metainformation | | | Wellenzahl [cm⁻¹] | | |
|---|---|---|---|---|---|
| **Produkt** | **Zeit (in Tagen)** | **Klima** | **11544** | **...weitere Wellenzahlen ...** | **3952** |
| Orangensaft | 4 | RT | 2,57869 | ... weitere Intensitätswerte... | 4,35357 |
| Orangensaft | 4 | RT | 2,6016 | ... weitere Intensitätswerte... | 4,35946 |

Gerade bei NIR-Spektren kann es ggf. gewünscht sein, nur bestimmte Wellenzahlenbereiche für die Auswertung heranzuziehen, so dass diese auf Wunsch herausgeschnitten und wieder zusammengefügt werden können.

Bei NIR-Spektren kann es im Laufe des Messvorgangs passieren, dass in bestimmten Wellenzahlbereichen die Empfindlichkeit des Detektors überschritten wird (beispielsweise bei wässrigen Proben im Bereich ca. 5400 cm⁻¹ bis 4900 cm⁻¹, der sehr sensitiv auf enthaltenes Wasser in der Probe reagiert). Da eine Detektorsättigung keine relevante Information enthält und die Auswertungsqualität kompromittieren könnte, können solche Bereiche auf Wunsch bewusst ausgeschnitten und von der weiteren Analyse exkludiert werden.

Bei NIR-Spektren kann es ggf. zu einem Basislinienshift in einzelnen Spektren kommen, falls es während der Messung zu einem Einfall von unerwünschtem Streulicht kam. Dies kann durch eine Streulichtkorrektur behoben werden. Eine Möglichkeit ist hierbei die sog. Vektornormierung (SNV-Normierung), die wie folgt funktioniert: Hierbei wird eine Probenmatrix P zunächst transponiert, anschließend wird für jeden Wert pro Spalte der korrigierte Wert nach der Formel

$$x' = \frac{x - \bar{x}}{\sigma}$$

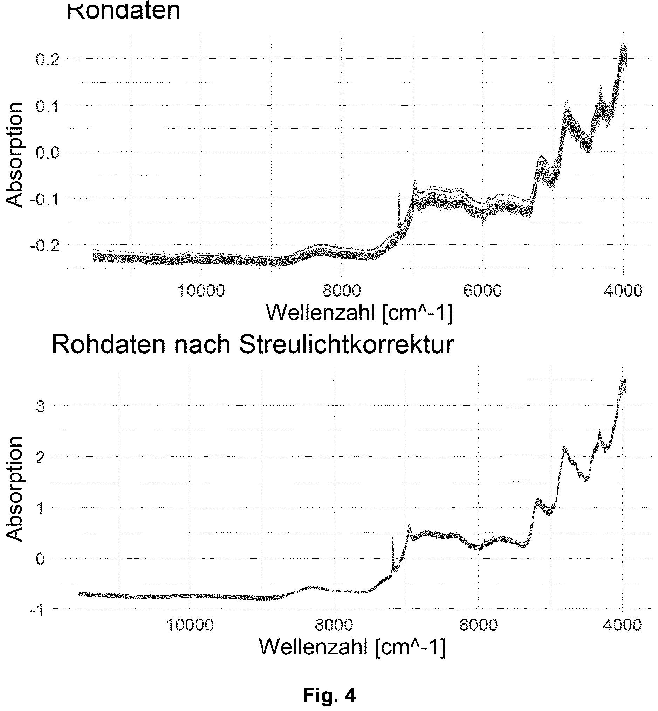

berechnet. Hierbei ist: x: Wert in einer Spalte, $\bar{x}$: Mittelwert der Spalte, $\sigma$: Standardabweichung der Spaltenwerte. Anschließend wird die resultierende Matrix zurück transponiert, so dass wieder in jeder Zeile eine Probe aufgeführt ist. Weiterhin kann für die Streulichtkorrektur im NIR-Bereich in Ausführungsformen der Erfindung beispielsweise auch eine Multiplikative Streulichtkorrektur (MSC) zum Einsatz kommen. Beide Vorgehensweisen sind allgemein bekannte Standardverfahren. In Fig. 4 ist ein exemplarischer NIR-Spektrenoverlay vor und nach Datenvorbehandlung gezeigt.

Weiterhin kann es ggf. vorteilhaft sein, NIR-Spektren (oder andere Rohdatenmatrices) ggf. vor der eigentlichen Analyse zu differenzieren und/oder einem Glättungsalgorithmus zu unterwerfen. Für diese Anforderungen wird der Savitzky-Golay-Algorithmus verwendet (vgl. Zitierstelle [16]), der beide Datenbehandlungen automatisch erledigt.

Sofern gewünscht kann vor der eigentlichen Distanzberechnung bereits eine Principal Component Analysis (PCA) der vorbereiteten Rohdaten erfolgen. Eine nachfolgende Distanzberechnung würde dann auf den ermittelten Scorewerten erfolgen. Dieser Schritt kann sinnvoll sein, wenn stark verrauschte Rohdaten verarbeitet werden müssen. Vor der PCA können die Daten außerdem zentriert, UV (Univarianz-)- oder Pareto-Skaliert werden (Zentrierung: $x' = x - \bar{x}$, UV-Skalierung: $x' = \frac{x - \bar{x}}{\sigma}$, Pareto-Skalierung: $x' = \frac{x - \bar{x}}{\sqrt{\sigma}}$, anders als bei der Streulichtkorrekur keine Transposition der Datenmatrix vor Berechnung).

Anschließend kann vom Nutzer das gewünschte Distanzmaß für die eigentliche Berechnung gewählt werden. Im Regelfall wird dies die euklidische Distanz sein, allerdings sind auch andere Distanzmaße denkbar, insbesondere die Manhattan-Distanz, Pearson-Distanz, Gower-Distanz oder die Mahalanobis-Distanz.

Nach Abschluss dieser Vorverarbeitung bestimmt die Applikation nun aus den Meta-Daten der Rohdaten für jede enthaltene Probe den Startwert bzw. den "Null-Wert" (mit anderen Worten: die Einzeldatenpunkte zum Zeitpunkt 0), d. h. den chemisch / physikalischen / biologischen Zustand der Probe, insbesondere -ohne darauf beschränkt zu sein- Zuckergehalt, Zerfallsgeschwindigkeit, Farbe, Bruchfestigkeit, Zerfallszeit, Friabilität, Dichte, Viskosität, Brechungsindex und/oder optischer Drehwinkel vor einer Einlagerung z.B. bei bestimmten klimatischen Bedingungen oder gezieltem Stresstest, wie beispielsweise Bestrahlung durch UV/-VIS-Licht, Forcieren von Redox-Reaktionen, o. ä.. Der Startwert oder Nullwert wird durch den Anwender definiert bzw. bestimmt. In den Metadaten der Proben ist dabei exakt gekennzeichnet, welche Proben bzw. welche Chargen der Proben zum Zeitpunkt 0 aufgenommen worden sind. Gibt es von einer Charge dabei mehrere Replikate des Zeitpunkts 0 so wird hiervon das Mittelwertspektrum berechnet und zur späteren Verwendung im Speicher abgelegt. Dieses so genannte Ausgangsdatum jeder entsprechenden Probe dient dann als Referenz, zu dem eine Distanzberechnung erfolgt.

Anschließend erfolgt die Distanzberechnung für jeden Zeitpunkt, wobei diese aus den Metadaten ermittelt sein kann, für jede Charge, bzw. für jeden Probenansatz nach der oben vom Nutzer gewählten Methode. Die so erhaltenen Distanzwerte können dann entsprechend sortiert, aggregiert und graphisch dargestellt werden, sowie statisch näher ausgewertet werden.

Aus den vorprozessierten Rohdaten wird in der beschriebenen Ausführungsform auch eine zusätzliche PCA berechnet, die nicht in die Distanzberechnung eingeht, sondern eine weitere, allerdings rein qualitative Interpretation der Rohdaten unterstützt.

ANWENDUNGSBEISPIEL 1 - NIR-Messungen eines flüssigen Lebensmittels: Orangensaft Gegenstand der Untersuchung waren:

- Orangensaft "Beste Wahl" von REWE (100% Saft aus Direktsaft, mit Fruchtfleisch, 8,5g Zucker auf 100ml (laut Etikett))

- Öffnung der Packung und Einlagerung eines Aliquots bei Raumtemperatur (22°C)

- Einlagerung eines weiteren Aliquots bei 40 °C, relative Luftfeuchte = 75 %

Als Messinstrument wurde ein Nahinfrarotspektroskopie-Gerät MPA II (Bruker Optik GmbH) verwendet:

- Messung des Orangensaftes in Transmission;

- Wellenzahlbereich: 12500 cm$^{-1}$ bis 3950 cm$^{-1}$ (spektrale Bereiche mit einer Absorption > 4,5 wurden nicht für die Auswertung berücksichtigt)

Datenvorbehandlung der NIR-Spektren:

- Bildung der 1. Ableitung mit 9 Glättungspunkten

Als Berechnungsparameter wurden die folgenden gewählt:

- Distanzmaß: Euklidische Distanz

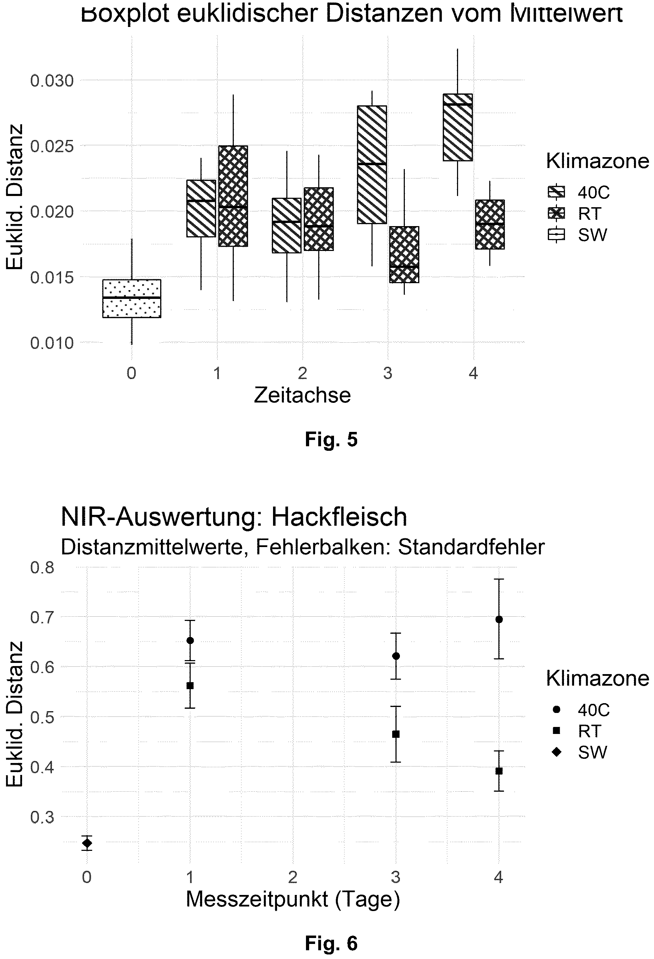

Fig. 5 zeigt den Boxplot der errechneten Distanzen über 5 Tage hinweg, wobei Tag 0 der "Startwert" (= "SW") ist. In den ersten beiden Tagen (Tag 1 und Tag 2) ist die Veränderung bei beiden Lagerbedingungen praktisch identisch und es besteht kein stat. signifikanter Unterschied. Ab Tag 3 wird jedoch deutlich, dass die ermittelte euklidische Distanz der Proben bei 40°C deutlich größer wird und auch an Tag 4 weiter zugenommen hat (Kruskal-Wallis-Test ergibt $p<0{,}05$, paarweiser Wilcox-Test ergibt statistische Signifikanz an Tag 3 und Tag 4 mit $p<0{,}05$, alle anderen Tage nicht signifikant). Dies deckt sich mit der allgemein zu erwartenden Annahme, dass eine Lagerung des Lebensmittels bei erhöhter Temperatur zu einer chemischen oder mikrobiellen Veränderung (z.B. Zersetzungsprozess einzelner Bestandteile oder Bakterienwachstum) führt.

ANWENDUNGSBEISPIEL 2 - NIR-Messungen eines festen Lebensmittels: Hackfleisch Gegenstand der Untersuchung waren:

- Frisch zubereitetes Schweine-Hackfleisch von REWE

- Einlagerung eines Aliquots unmittelbar nach Kauf bei Raumtemperatur (22°C)

- Einlagerung eines weiteren Aliquots bei 40°C, relative Luftfeuchte = 75 %

Als Messinstrument wurde ein Nahinfrarotspektroskopie-Gerät MPA II (Bruker Optik GmbH) verwendet:

- Messen der NIR-Spektren in diffuser Reflexion als Absorptionsspektren

- Wellenzahlbereich: 12500 cm$^{-1}$ bis 3950 cm$^{-1}$

Datenvorbehandlung der NIR-Spektren: Durchführung einer SNV-Streulichtkorrektur

Als Berechnungsparameter wurden die folgenden gewählt: Euklidische Distanz

In Fig. 6 sind die Mittelwerte (Fehlerbalken: Standardfehler) der errechneten euklidischen Distanzen aufgeführt. Die größte Veränderung erfolgt in allen Fällen von Tag 0 auf Tag 1. Generell ist zu sehen, dass sich das Hackfleisch wie erwartet bei höheren Temperaturen auch stärker verändert als bei Raumtemperatur (alle Zeitpunkte unterscheiden sich stat. signifikant mit $p<0{,}05$ (Kruskal-Wallis-, bzw. Wilcoxon Rangsummentest)) und auch die Plateaubildung bei den 40°C-Proben noch nicht abgeschlossen ist.

Parallel zu den genannten Messungen wurde eine Fotodokumentation (Fig. 7) durchgeführt (Start und Ende Beobachtungszeitraum). Bei der Probe, die bei erhöhter Temperatur gelagert wurde, ist ein deutlich verfärbter "Ring" (markiert mit (1)) zu sehen, zudem hatte sich eine Flüssigkeit oben auf der Fleischprobe gebildet, die bei der Raumtemperaturprobe nicht existierte. Zudem hatte sich die Textur der Probe (klare Abtrennung von Fleischanteil (markiert mit (2)) & Fett (markiert mit (3))) stark verändert, was bei der Vergleichsprobe deutlich geringer ausgeprägt war. Diese Veränderungen legen ein Verderben des Lebensmittels nahe.

ANWENDUNGSBEISPIEL 3 - Online-NIR-Messungen einer Extraktion von Eisenkraut Gegenstand der Untersuchung waren:

- 150 g geschnittenes Eisenkraut (schließt alle getrockneten oberirdisch wachsenden Pflanzenteile ein)

- Extraktion in einem Becherglas unter Rühren bei 20 °C in 1800 ml demineralisiertem Wasser

- Als Messinstrument wurde ein Nahinfrarotspektroskopie-Gerät MPA II (Bruker Optik GmbH) verwendet. Die zu vermessenden Probenzüge wurden vor der Messung filtriert.

- Wellenzahlbereich: 12500 cm$^{-1}$ bis 3950 cm$^{-1}$ (spektrale Bereiche mit einer Absorption > 4,5 wurden nicht für die Auswertung berücksichtigt)

Datenvorbehandlung der NIR-Spektren: Durchführung einer SNV-Streulichtkorrektur

Als Berechnungsparameter wurden die folgenden gewählt: Euklidische Distanz

Fig. 8 zeigt den Boxplot der mit dem neuen Verfahren berechneten Distanzen über einen Zeitraum von 0 bis 180 Minuten des Extraktionsvorgangs. Es ist deutlich, dass die Veränderung der Lösung mit zunehmendem Fortschreiten des Extraktionsvorgangs am Beginn des Experiments größer ist und gegen Ende sich langsam ein Plateauwert einstellt. Das Entscheidende, Neue des erfindungsgemäßen Berechnungsverfahrens ist allerdings, dass in diesem Experiment nicht eine *einzige* Leitsubstanz stellvertretend für den ganzen Extraktionsvorgang verfolgt wird, sondern die Gesamtheit der gelösten Substanzen über das komplette NIR-Spektrum verfolgt wird. Verschiedene chemische Pflanzenbestandteile können eine unterschiedliche Extaktionsgeschwindigkeit / Löslichkeitskinetik aufweisen. Wird nur eine einzige Leitsubstanz für die Beurteilung einer "erschöpfenden" Extraktion herangezogen, so könnte es sein, dass genau diese Substanz in ihrer Lösungskinetik nicht repräsentativ für alle enthaltenen Stoffe ist. Mithin könnte so eventuell eine Extraktion vorzeitig beendet werden, weil diese Leitsubstanz nach einem Zeitraum keine Veränderung der Lösung mehr anzeigt, obgleich andere, nicht erfasste Bestandteile durchaus noch nicht vollständig in die Lösung übergegangen sind. Das vorliegende Verfahren beseitigt dieses Problem, da hier *alle* Substanzen der Lösung erfasst werden und somit auch eine "Gesamtkinetik" aller Lösungsvorgänge erfasst werden kann.

Die Erfassung einer von Produktionscharge zu Produktionscharge identischen Extraktion ist dabei insbesondere bei der Herstellung pharmazeutischer Extrakte von besonderer Bedeutung, da der Wirkstoffgehalt in den hergestellten Produkten stets identisch sein soll. Schwankende Wirkstoffgehalte können in einer späteren Qualitätskontrolle zu "out-of-specification"-Ereignissen führen, deren Konsequenz ggf. eine Verwerfung einer Produktionscharge sein kann. Die erfindungsgemäße Methode kann dazu beitragen, mit geringem Aufwand diese Anforderung signifikant zu erleichtern, bzw. überhaupt erst zu ermöglichen.

Fig. 9 zeigt die aus den Rohdaten von Fig. 8 errechnete Gesamtkinetik. Dargestellt sind als Punkte die Mittelwerte mit Standardfehler aus den Einzelmessungen des Boxplots (aus Fig. 8), sowie eine nachträglich errechnete,

$$Distanz(t) = \frac{Distanz_{max} * t}{k + t}$$

nicht-lineare Kinetik-Kurve. Für diese wurde die Formel genutzt. Ebenso hätte eine andere, adäquate mathematische Beschreibung genutzt werden können. Dabei ist *Distanz(t)* = Distanz zum Zeitpunkt *t*; *Distanz$_{max}$* = Distanzwert im Plateau; k = Geschwindigkeitskonstante.

Aus der in diesem Beispiel dargestellten Kinetik für das durchgeführte Experiment kann außerdem exemplarisch abgeleitet werden, dass die genutzte Pflanzenprobe nach ca. 4 Minuten zu 50% extrahiert wurde, eine 90%ige Extraktion nach ca. 35 Minuten erreicht wurde und eine nahezu erschöpfende Extraktion mit 95% nach etwa 75 Minuten abgeschlossen ist. Dies bedeutet auch, dass in hier nach 75 min die Extraktion beendet werden kann und beispielsweise nicht unnötigerweise noch weitere Stunden fortgeführt werden muss, was im Industriemaßstab mit erheblichen Kosten verbunden wäre.

ANWENDUNGSBEISPIEL 4 - Verfolgung des Einflusses von Stressfaktoren auf Zellkulturen Vor dem unten beschriebenen Hauptversuch wurde ein Vorversuch durchgeführt, der zeigen sollte, ob HEK295-Zellen

a) in NIR-Messgefäßen konfluent wachsen können

b) von Ethanol und gegebenenfalls in welchen Konzentrationen gestresst werden können, so dass deren Viabilität / Konfluenz entsprechend abnimmt. Dies wurde insbesondere mit der aufwändigen, aber bewährten Trypanblaufärbung verifiziert. Hierfür wurden Konzentrationen von 0 - 10 % Ethanol getestet.

Ausgehend von den Ergebnissen dieses Vorversuchs wurde beschlossen, zwei verschiedene Konzentrationen von Ethanol im Hauptversuch zu verwenden, da hier ein klarer Einfluss auf die Zellviabilität zu sehen war in einem Zeitraum, der sich später technisch leicht abdecken ließ.

Im Anschluss wurde der Hauptversuch durchgeführt, der die Abnahme der Viabilität / Konfluenz durch eine NIR-Messung unter Anwendung des erfindungsgemäßen Rechenverfahrens bestätigte.

Gegenstand der Untersuchung waren:

- HEK295 Zellen, bereitgestellt von Fa. Microbify, in einer Konzentration von $2,2 * 10^5$ Zellen/ml in Medium, konfluent gewachsen, d.h. adhärent gewachsene Zellen, die einen durchgängigen Monolayer / Zellrasen ausgebildet haben, in den NIR - Messgefäßen mit Transflexionsstempel aus Edelstahl von BrukerOptics

  ◦ Nutzung von Nährmedium DMEM (Dulbecco's Modified Eagle's Medium), mit hohem Glukosegehalt, HEPES-Puffer, ohne Phenolrot-Indikator. Medium wurde gebrauchsfertig von Fa. ThermoFisher, Katalognummer 21063045, bezogen und mit mit 10% fetalem Kälberserum (v/v) und 1% Penicillin-Strepomycin-Lösung (v/v) versetzt.

  ◦ Zellanzucht für 48h vor Messung direkt im Messgefäß

- Zugabe mehrerer Konzentrationen an Ethanol: 0% (Kontrolle), 3%, 6% (jeweils v/v). Diese EtOH - Konzentrationen wurden aufgrund des Vorversuchs, der in einem Zellviabilitätsassay eine entsprechende Absterbe / Ablösungs-Reaktion hervorrief ausgewählt. Vor Beginn des Experiments waren die Zellen konfluent, d.h. zu 100% viabel

- Messung der Zellveränderung via NIR über einen Zeitraum von 380 min

Als Messinstrument wurde ein Nahinfrarotspektroskopie-Gerät microPHAZIR GP (Thermo Fisher Scientific Inc.) verwendet mit einem Wellenzahlbereich: 6266 cm$^{-1}$ bis 4172 cm$^{-1}$ Datenvorbehandlung der NIR-Spektren: Keine besondere Vorbehandlung erfolgt, Spektren wurden direkt weiterverarbeitet.

Als Berechnungsparameter wurden die folgenden gewählt: Euklidische Distanz

Die Zellen wurden aus dem Inkubator entnommen und mit einem chemischen Stressfaktor (Ethanol), in den genannten Konzentrationen versetzt. Hierbei wurden jeweils 3 unabhängige Replikate mit 0%, 3% und 6% Ethanol angesetzt und in einem engen zeitlichen Abstand (alle 2 Minuten) über einen Zeitraum von insgesamt 6 Stunden via NIR vermessen.

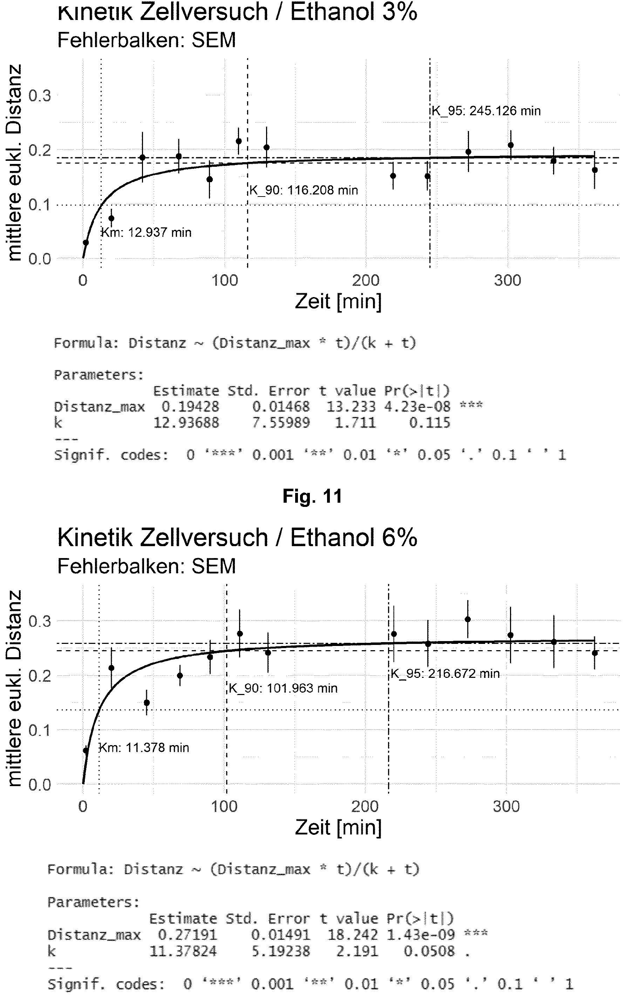

Aus den gemessenen Spektren wurden gemäß dem erfindungsgemäßen Verfahren die euklidischen Distanzen errechnet, pro Messzeitpunkt gemittelt und die Anpassung von Kinetik-Kurven (vgl. Anwendungsbeispiel 3; identische Vorgehensweise) durchgeführt. Diese sind in Fig. 10, 11 und 12 dargestellt, jeweils für Kontrolle, Behandlung mit 3% Ethanol und 6% Ethanol. Die Datenpunkte sind jeweils die Mittelwerte aus 3 unabhängigen Replikaten, die jeweils 3-mal gemessen wurden, somit handelt es sich um 9 Messpunkte pro Punkt. Bei den Fehlerbalken handelt es sich um den Standardfehler des Mittelwerts. Eingezeichnet sind außerdem die Ermittlungspunkte für $K_m$ (Zeitpunkt, an der 50% des Plateauwerts erreicht wurde), $K_{90}$ (Zeitpunkt, an dem 90% des Plateauwerts erreicht wurde), sowie $K_{95}$ (Zeitpunkt, an dem 95% des Plateauwerts erreicht wurde).

Diese Werte werden in der Kontrolle sehr schnell erreicht ($K_m \sim 2,4$ min, $K_{95} \sim 43,7$ min), wobei der Plateauwert an sich auch den geringsten Wert innerhalb der Experimente einnimmt. Offensichtlich ist auch das Entnehmen der Zellen aus dem Inkubator und die Verwendung auf der Laborbank bereits ein geringer Stressfaktor. Die $K_m$- Werte der ethanolisch gestressten Zellen sind statistisch identisch (bei 3% EtOH - 13min, bei 6% EtOH - 11min), was auf eine einheitliche, konzentrationsunabhängige Initialreaktion der Zellen auf die Ethanolzugabe zurückzuführen sein könnte.

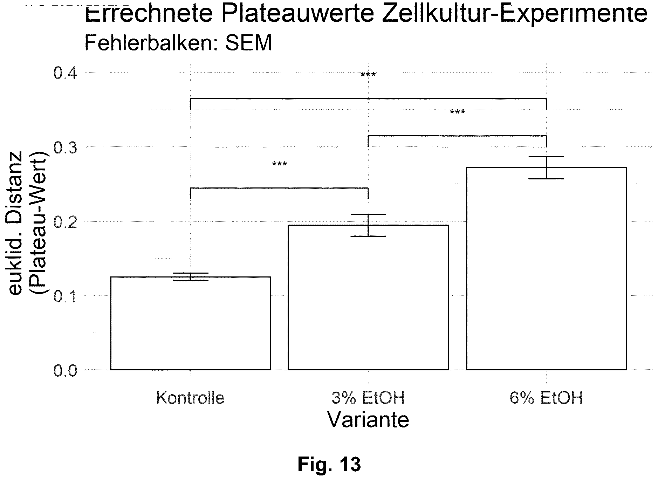

Bei der Zugabe von Ethanol in verschiedenen Konzentrationen wird allerdings sukzessive ein höheres Plateau erreicht, was auf eine deutlich höhere Stressbelastung der Zellen hinweist. Hierzu passt auch, dass sich die Zellen in den Messgefäßen (wie auch schon im Vorversuch beobachtet) im Laufe der Zeit von der Oberfläche lösten und ihre Konfluenz aufgaben. Dies ist auch eindeutig aus dem Balkendiagramm (Fig. 13) ersichtlich (statistische Signifikanz, One-way ANOVA mit p<0,001; post-hoc-Test: paarweiser t-test, alle p-Werte < 0,001), welches aus den Parametern der Kurvenanpassungen aus Fig. 10, 11 und 12 generiert wurde.

Auch hier ist die entscheidende Neuheit, dass für den Nachweis einer Zell-Stressung nicht erst eine stellvertretende Einzelsubstanz aus der Zellsuspension identifiziert, isoliert und quantifiziert werden müsste oder aufwändige Zell-Färbungs- und Auszähl-Assays etabliert und durchgeführt werden müssen, die zudem beispielsweise nicht als Online-Messung gestaltet werden können. Stattdessen kann ein sehr hohes Probenaufkommen ohne komplexe externe Probenvorbereitung direkt spektroskopisch vermessen und mit geringem Aufwand durch das erfindungsmäße Verfahren ausgewertet werden. Zudem wurde hier der Nachweis erbracht, dass das vorliegende Verfahren auch bei der Verfolgung der Stabilität / Viabilität lebender Zellen erfolgreich eingesetzt werden kann.

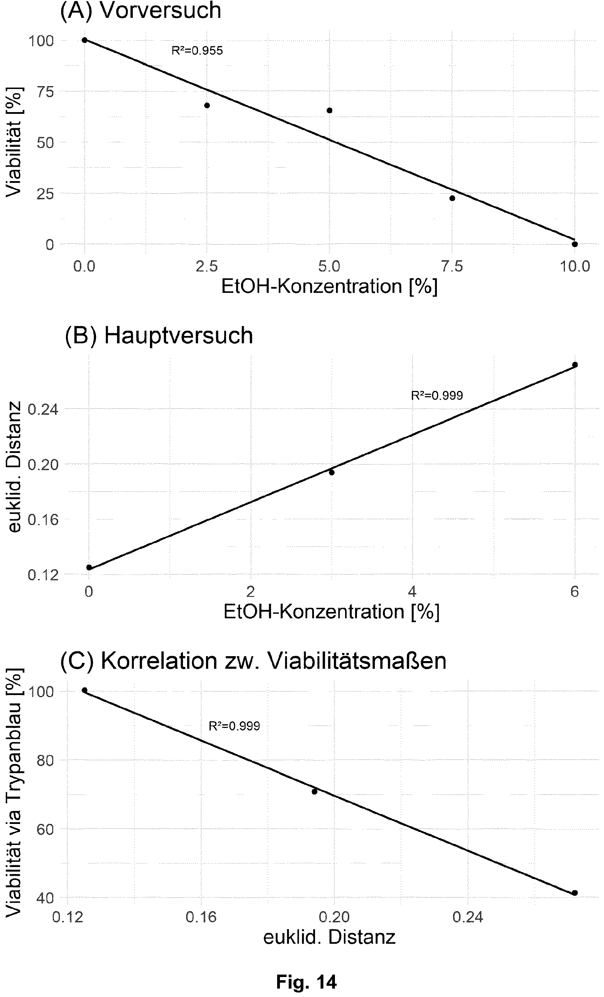

Die beobachteten Zellviabilitäten am Ende des Vorversuchs (die aufgrund leicht unterschiedlicher, eingesetzter EtOH-Konzentrationen linear interpoliert wurden) korrelieren mit den beobachteten maximalen euklidischen Distanzen (mit $R^2 > 95\%$) (siehe Fig. 14, sowie die im folgenden abgebildete Tabelle).

Tabelle 2: Gegenüberstellung der Distanzmaße berechnet als Euklidische Distanz mit den Resultaten eines kolorimetrischen Zellviabilitätsassays (Färbung mit Trypanblau).

|  | Vorversuch: Zellviabilität | Hauptversuch: maximale euklidische Distanz |
|---|---|---|
| 0% Ethanol | 100% | 0,125 |
| 2,5% Ethanol | 68% | (nicht gemessen) |
| 3% Ethanol | 70,84% (interpoliert) | 0,194 |
| 5% Ethanol | 65,5% | (nicht gemessen) |
| 6% Ethanol | 41,38% (interpoliert) | 0,272 |
| 7,5% Ethanol | 22,5% | (nicht gemessen) |
| 10% Ethanol | 0% | (nicht gemessen) |

ANWENDUNGSBEISPIEL 5 - Vergleich eines Arzneimittels (Arzneimittel-Tee) mit einer photometrischen Referenzmethode

Gegenstand der Untersuchung waren:

- Arzneimitteltee: Bad Heilbrunner Leber- und Gallen-Tee (1 Filterbeutel mit 1,75 g enthält laut Hersteller: 0,61 g Pfefferminzblätter, 0,35 g Löwenzahn, 0,26 g Javanische Gelbwurz, 0,18 Schafgarbenkraut; ohne Mengenangabe: Fenchel, Kamillenblüten, Kümmel, Süßholzwurzel)

- Extraktion von 3 Teebeuteln in einem Becherglas unter Rückfluss (Siedetemperatur Wasser, ca. 100 °C) in 150 ml demineralisiertem Wasser

- Als Messinstrument wurde ein Nahinfrarotspektroskopie-Gerät MPA II (Bruker Optik GmbH) verwendet. Die zu vermessenden Probenzüge wurden vor der Messung filtriert.

- Wellenzahlbereich: 12500 cm$^{-1}$ bis 3950 cm$^{-1}$ (spektrale Bereiche mit einer Absorption > 4,5 wurden nicht für die Auswertung berücksichtigt)

Datenvorbehandlung der NIR-Spektren: Durchführung einer SNV-Streulichtkorrektur

Als Berechnungsparameter wurden die folgenden gewählt: Euklidische Distanz

Als Referenzmethode kam eine validierte, photometrische Methode zum Nachweis von Gesamtpolyphenolen (Messung der Absorption bei 760nm nach Umsetzung der Lösung mit einem Molybdat-Wolframat-Reagenz-Reagenz und 29%iger Natriumcarbonatlösung, in Anlehnung an die Monographie 2.8.14 des europäischen Arzneibuchs) zum Einsatz.

Ziel der Untersuchung war die Frage, ob eine spektroskopische Analyse der Teeproben am Zeitpunkt 0 und 10 Tage bei Lagerung der Probe bei 4°C im Kühlschrank (KS), bei 20°C (Raumtemperatur, RT) und bei 40°C eine ähnliche Wiederfindungsrate / Stabilität nachweisen kann, wie eine validierte, photometrische Referenzmethode, die Gesamtpolyphenole (GPP) einer Lösung nachweist.

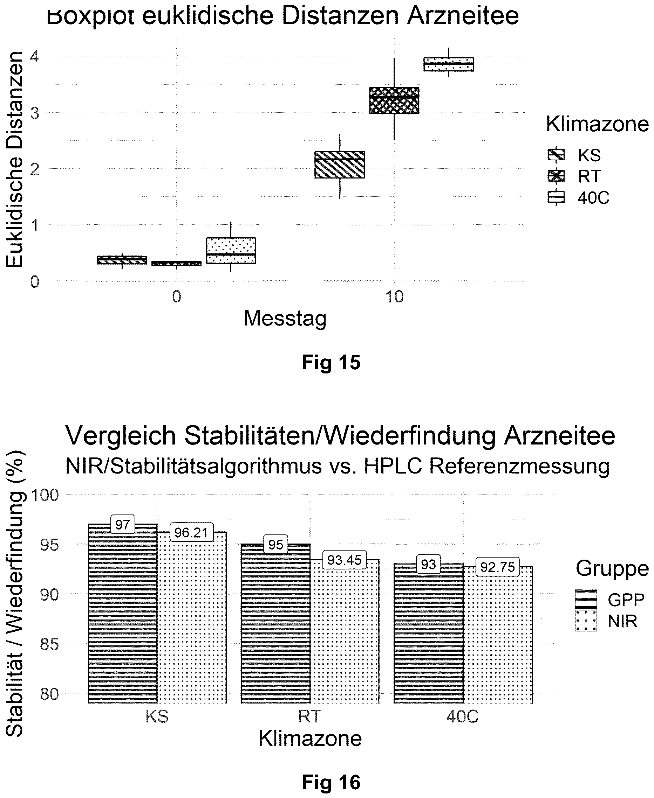

Fig. 15 zeigt die ermittelten euklidischen Distanzen aus den NIR-Messungen am Tag 0 und Tag 10. Diese nehmen bei den Proben im Kühlschrank (KS) am geringsten zu, bei Lagerung bei Raumtemperatur (RT) moderat und bei 40°C am stärksten, was intuitiv zu erwarten ist.

Der Vergleich mit den Wiederfindungsraten bzw. Stabilität aus der photometrischen Methode (in Legende bezeichnet mit "GPP") in Fig. 16 zeigt außerdem, dass die neue Berechnung über Distanzmaße gut mit dieser Referenz übereinstimmt. Dass sie etwas niedriger liegt als in der Referenzmethode ist nachvollziehbar, da das neue Verfahren *alle* Substanzen im Stoffgemisch erfasst, die Referenzmethode jedoch nur eine Teilmenge.

Für die Berechnung einer prozentualen Stabilitätsrate wurden für die NIR-Daten ein hypothetisches Leerwert-Spektrum ohne Analyten angenommen und dessen euklidische Distanz zum gemittelten Ausgangsspektrum berechnet. Diese Distanz wurde dann genutzt, um alle anderen Distanzen in diesem Intervall zu normieren. Für die Referenzmethode wurden die Gesamtpolyphenole in mg/100g bestimmt und deren Abbau nach 10 Tagen entsprechend ebenfalls auf einen prozentualen Wert normiert.

ANWENDUNGSBEISPIEL 6 - VERGLEICH EINER AUSZUGSMISCHUNG IN VERSCHIEDENEN KLIMAZONEN MITTELS HPLC/MS

Gegenstände der Untersuchung waren:

- Ethanolisch-wässriger Auszug einer Arzneipflanzenmischung aus Thymian, Rosmarin und Kamille (1:1:1) mittels einer 20%-igen ethanolisch wässrigen Mischung

- Lagerbedingungen:

  AC (Accelerated conditions): Temperatur = 40 °C, relative Luftfeuchte = 75 %

  KII (Klimazone II): Temperatur = 25°C, relative Luftfeuchte = 60%

  Einlagerung für jeweils 24 Wochen

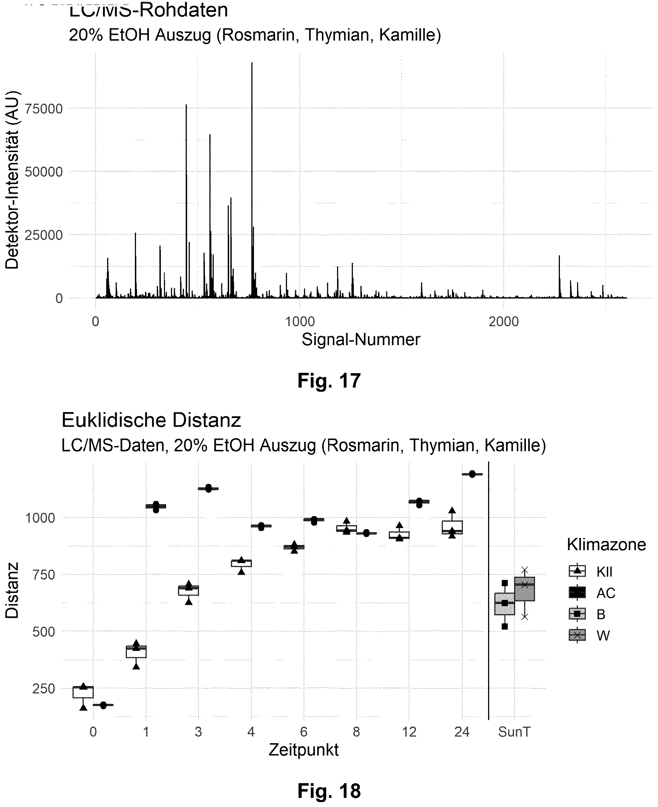

- Eine zusätzliche, unabhängige Probe: Bestrahlung für 20 Stunden bei 150 klx im Sun Tester in Braunglas- / Weißglas-Fläschchen (in Abbildungslegende von Fig. 18 mit "B", bzw. "W" bezeichnet)

Verwendetes Gerät: Suntest CPS+ (Atlas Material Testing Technology GmbH)

- Wellenlänge: 320 nm bis 800 nm

- Bestrahlungsdosis: 3000 klx*h

Als Messinstrumente wurden folgende Geräte genutzt:

- Chromatographisches System: 1290 Infinity II (Agilent Technologies Germany GmbH & Co. KG)

- Detektor: TripleTOF (Sciex)

Die folgenden Berechnungsparameter wurden gewählt:

- Distanzmaß: Euklidische Distanzen

- Berechnung auf LC/MS-Peaktable (2602 Einzelsignale)

- Vorverarbeitung: Rechnung auf latenten Variablen, Varianzabdeckung > 95%

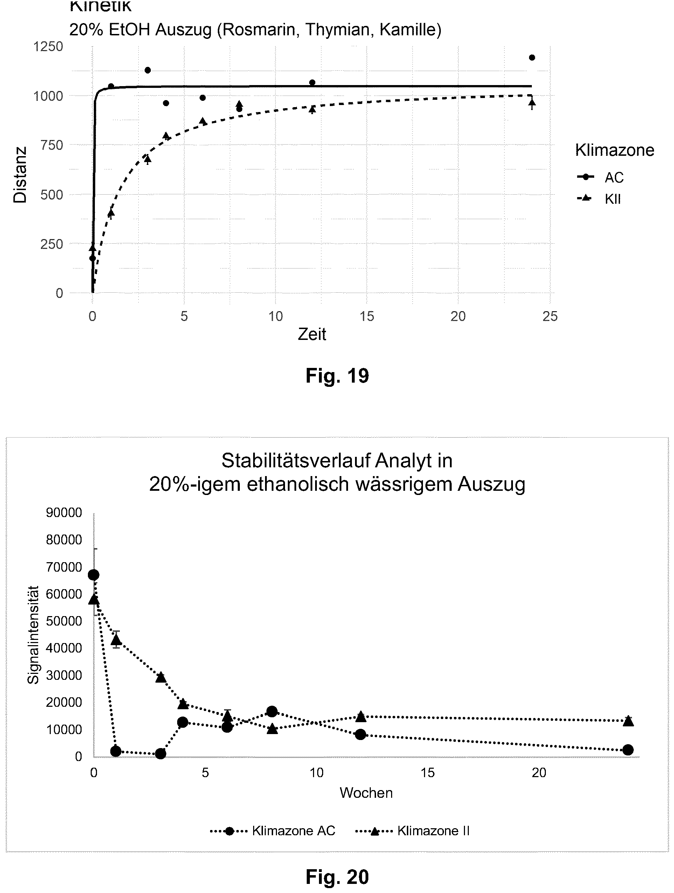

Für die Kinetik-Berechnungen (Fig. 19) wurde stets die Gleichung $Distanz = \frac{Distanz_{max} * t}{k + t}$ verwendet und $Distanz_{max}$, sowie $k$ rechnerisch bestimmt.

Die Ergebnisse der Untersuchung wird in Abbildung 19 dargestellt. Deutlich zu erkennen ist, dass in der strengeren Klimabedingung ("AC") die Veränderungen sehr viel schneller eintreten, da früher eine höhere Distanz erreicht wird. Allerdings erreichen die Proben in beiden Klimabedingungen praktisch das gleiche Distanzplateau gegen Ende der Untersuchung.

Mit der Bestrahlung der Proben im Suntester (siehe Fig. 18) wird innerhalb von ca. 20 Stunden das gleiche Veränderungsniveau erreicht, dass sonst erst nach ca. 3,5 Wochen Einlagerung unter Klimazone II - Bedingungen erzielt werden. Es gibt dabei keinen signifikanten Unterschied zwischen Braun- und Weißglasfläschchen.

Durch eine klassische Herangehensweise mittels einer stellvertretenden Markersubstanz (m/z 329,17; Retentionszeit = 8,16 min, Fig. 20) zeigt sich wie bei der NIR-Auswertung ein erwartbarer Abbau der Verbindung über die Zeit, der bei Bedingung AC deutlich schneller den Endpunkt erreicht als bei Bedingung Klimazone II. Im Gegensatz zur Verarbeitung eines Datensatzes, der die Information vieler Verbindungen enthält (wie hier NIR-Spektren) zeigt dieser analytische Ansatz mit lediglich einer einzigen Markersubstanz nur ein sehr limitiertes Bild dieses Produkts und kann das gesamte Vielstoffgemisch nicht wirklich repräsentieren. Zudem ist die Auswertung von Datensätzen bzgl. Einzelverbindungen deutlich aufwändiger und zeitintensiver.

ANWENDUNGSBEISPIEL 7 - VERGLEICH VON PACKMITTELN MITTELS NIR UNTER EINFLUSS EINER BESTRAHLUNG MIT INTENSIVEM LICHT UND ERHÖHTER TEMPERATUR

Gegenstände der Untersuchung waren:

-  Eine Arzneidrogen-Pulvermischung aus Rosmarin, Thymian und Kamille im Verhältnis 1:1:1

-  Lagerbedingungen: geschlossen in einer Papiertüte (="P1") bzw. offen gelagert (="P2")

Eine Bestrahlung mit Licht erfolgte unter folgenden Bedingungen:

-  Gerät: Suntest CPS+ (Atlas Material Testing Technology GmbH)

-  Wellenlänge: 320 nm bis 800 nm

-  Bestrahlungsdosis: 3000 klx*h

Als Messinstrument wurde ein Nahinfrarotspektroskopie-Gerät MPA II (Bruker Optik GmbH) verwendet:

-  Messung in diffuser Reflexion

Als Berechnungsparameter wurden die folgenden gewählt:

-  Distanzmaß: Euklidische Distanz

-  Berechnung auf SNV-korrigierten NIR-Spektren ohne jede Einschränkung von Wellenzahlbereichen

-  Distanzberechnung auf latenten Variablen mit abgedeckter Gesamtvarianz > 95%

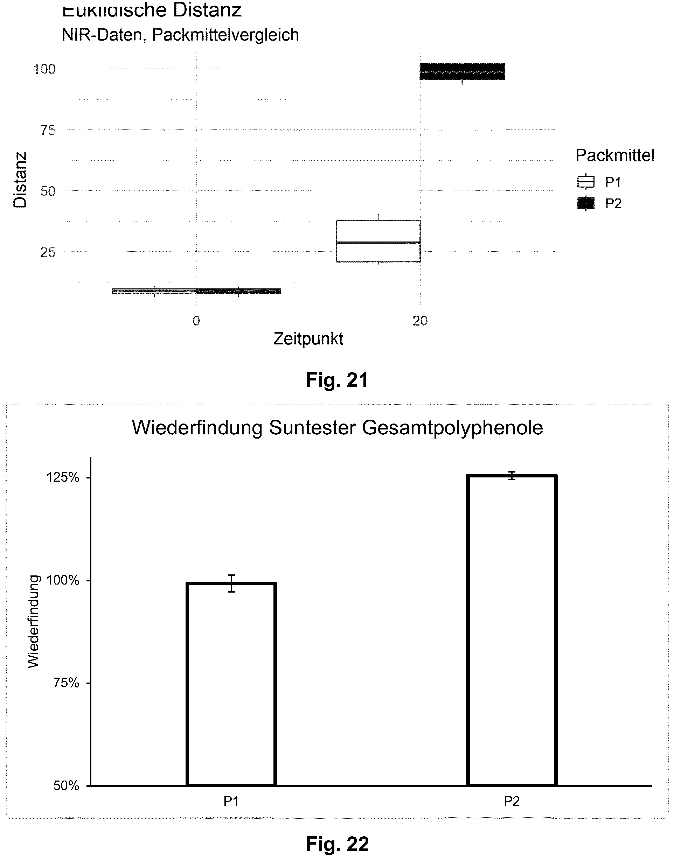

Als Ergebnis der Untersuchung wurde folgendes festgehalten:
Das Ergebnis ist Fig. 21 in Form eines Boxplots dargestellt. Zum Zeitpunkt 0 (vor Start der Bestrahlung im Sun Tester sind die Distanz Werte in beiden Proben identisch, jedoch sind die Werte zum Zeitpunkt 20 Stunden nicht nur vom Startwert unterschiedlich, sondern auch P1 (Pulvermischung in Papiertüte) und P2 (offen gelagerte Pulvermischung) sind signifikant voneinander zu unterscheiden. Dies ist einerseits auf die starke Lichteinwirkung zurückzuführen (daher der starke Anstieg in Probe P2), aber auch auf die erhöhte Temperatur, die hier im Sun Tester erreicht wird und auch die Probe P1 (in der Papiertüte) beeinflusst. Diese Resultate wurden auch durch ein klassisches Analyseverfahren bestätigt. Dazu wurden die Gesamtpolyphenole in Anlehnung an die allgemeine Monographie 2.8.14 des Europäischen Arzneibuchs photometrisch erfasst. Dabei handelt es sich um ein sehr aufwändiges nasschemisches Analyseverfahren. Auch hier zeigte die Probe P2 eine deutliche Abweichung zum Ausgangswert (Unterschied zum Ausgangswert= 26 %), wohingegen die Probe P1 (Unterschied zum Ausgangswert = 1 %) in der Papiertüte nahezu unverändert blieb (siehe Fig. 22).
Durch das erfindungsgemäße Verfahren wird also eindrücklich deutlich, dass Temperatur und Lichteinwirkung zusammen einen messbaren Einfluss gegenüber nur einer thermischen Einwirkung, bzw. keiner Einwirkung der beiden Faktoren hat. Die sehr zeitaufwändige nasschemische Analyse der Polyphenole bestätigt das Auftreten von Veränderungen der Probe, kann aber eben nur einen Teil dieser Veränderungen abbilden, da Polyphenole nur ein Ausschnitt des gesamten chemischen Profils darstellen. Das erfindungsgemäße Verfahren erfasst dagegen das komplette Profil und zeigt daher auch ein viel umfassenderes Bild.

ANWENDUNGSBEISPIEL 8 - VERGLEICH VERSCHIEDENER KLIMAZONEN MITTELS NIR

Gegenstände der Untersuchung waren:

-  Eine Arzneidrogen-Pulvermischung aus Rosmarin, Thymian und Kamille im Verhältnis 1:1:1

Lagerbedingungen:

AC (Accelerated conditions): Temperatur = 40 °C, relative Luftfeuchte = 75 %

KII (Klimazone II): Temperatur = 25°C, relative Luftfeuchte = 60%

Einlagerung für jeweils 24 Wochen

Als Messinstrument wurde ein Nahinfrarotspektroskopie-Gerät MPA II (Bruker Optik GmbH) verwendet:

- Messung in diffuser Reflexion

Als Berechnungsparameter wurden die folgenden gewählt:

- Distanzmaß: Euklidische Distanz

- Berechnung auf SNV-korrigierten NIR-Spektren ohne jede Einschränkung von Wellenzahlbereichen

- Distanzberechnung auf latenten Variablen mit abgedeckter Gesamtvarianz > 95%

Als Messinstrumente für den parallelen, klassischen analytischen Ansatz wurden folgende Geräte genutzt:

- Chromatographisches System: 1290 Infinity II (Agilent Technologies Germany GmbH & Co. KG)

- Detektor: TripleTOF (Sciex)

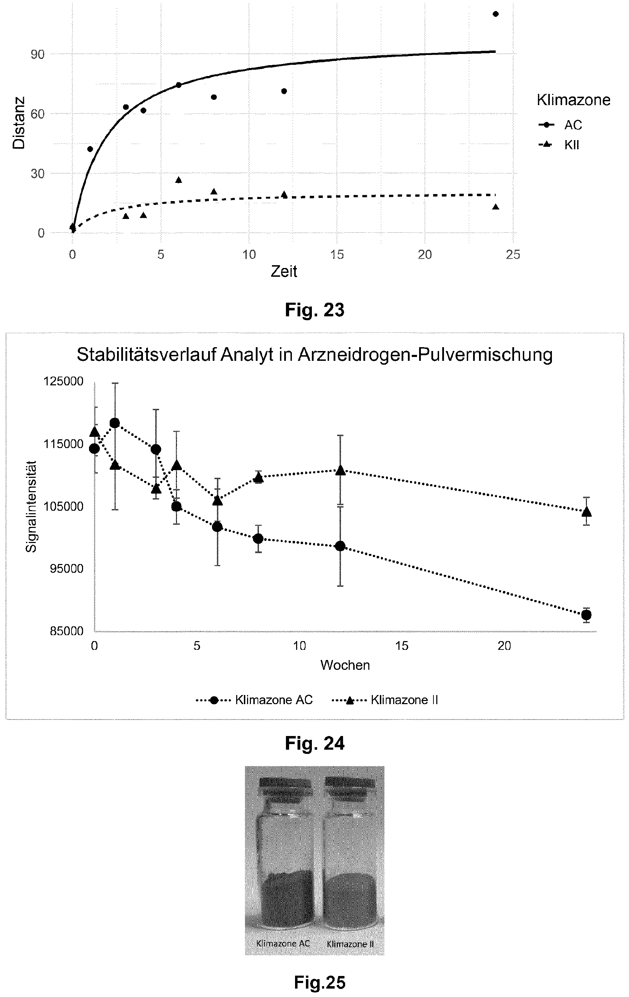

Als Ergebnis der Untersuchung wurde folgendes festgehalten:
Aus Abbildung 23 ist sofort ersichtlich, dass die strengere Klimazone AC bereits in der ersten Woche zu signifikant höheren Distanzen gegenüber der Klimazone II führen. Dies entspricht natürlich den Erwartungen, die parallel auch über einen klassischen analytischen Ansatz über HPLC/MS über eine einzige stellvertretende Verbindung abgebildet wird. Dazu ist in Fig. 24 der Verlauf der Signalintensität einer Hauptverbindung ($m/z = 329,17$; Retentionszeit $= 8,16$ min) aus der Pulvermischung gezeigt. Hier zeigt sich bei Klima AC ein deutlich schnellerer Abbau als bei Klimazone II. Jedoch wird dieser klassische Ansatz mit Betrachtung nur einer einzigen Substanz der Komplexität eines pflanzlichen Produkts nicht gerecht. Durch den neuartigen Ansatz wird ein großer Datensatz zu einem komplexen Produkt in seiner Gesamtheit betrachtet. Analog zu Beispiel 1 wurde auch hier eine Kinetik für die zwei verschiedenen Klimazonen über die ermittelten Distanzen aus dem NIR-Datensatz berechnet.

Die Veränderung nach 24 Wochen wird auch optisch offensichtlich (Fig. 25), bei der das Pulver im Probengläschen der Klimazone AC deutlich dunkler gefärbt ist als das Pendant der Klimazone II. Diese rein optische Beurteilung ist aber sehr subjektiv und generell schwer zu erfassen. Unser Rechenverfahren dagegen macht diese Änderung aber nicht nur qualitativ, sondern insbesondere quantitativ erfassbar.

ANWENDUNGSBEISPIEL 9 - VERGLEICH VON PACKMITTELN EINER FESTEN ARZNEIFORM MITTELS NIR

Gegenstände der Untersuchung waren:

- Weißdorn Filmtabletten:

    Jede Filmtablette enthält 450 mg Trockenextrakt aus Weißdornblättern mit Blüten

    Die Filmtabletten wurden zum einen im Blister in einer Faltschachtel eingelagert und zum anderen in Polyethylen-Beuteln abgefüllt eingelagert

Lagerbedingungen:

AC (Accelerated conditions): Temperatur = 40 °C, relative Luftfeuchte = 75 %

KII (Klimazone II): Temperatur = 25°C, relative Luftfeuchte = 60%

Einlagerung für jeweils 24 Wochen

Als Messinstrument wurde ein Nahinfrarotspektroskopie-Gerät MPA II (Bruker Optik GmbH) verwendet:

- Messung in diffuser Reflexion

Als Berechnungsparameter wurden die folgenden gewählt:

- Distanzmaß: Euklidische Distanz

- Berechnung auf SNV-korrigierten NIR-Spektren ohne jede Einschränkung von Wellenzahlbereichen

Als Messinstrumente für den parallelen, klassischen, analytischen Ansatz wurden folgende Geräte genutzt:

- Chromatographisches System: 1290 Infinity II (Agilent Technologies Germany GmbH & Co. KG)
- Detektor: TripleTOF (Sciex)

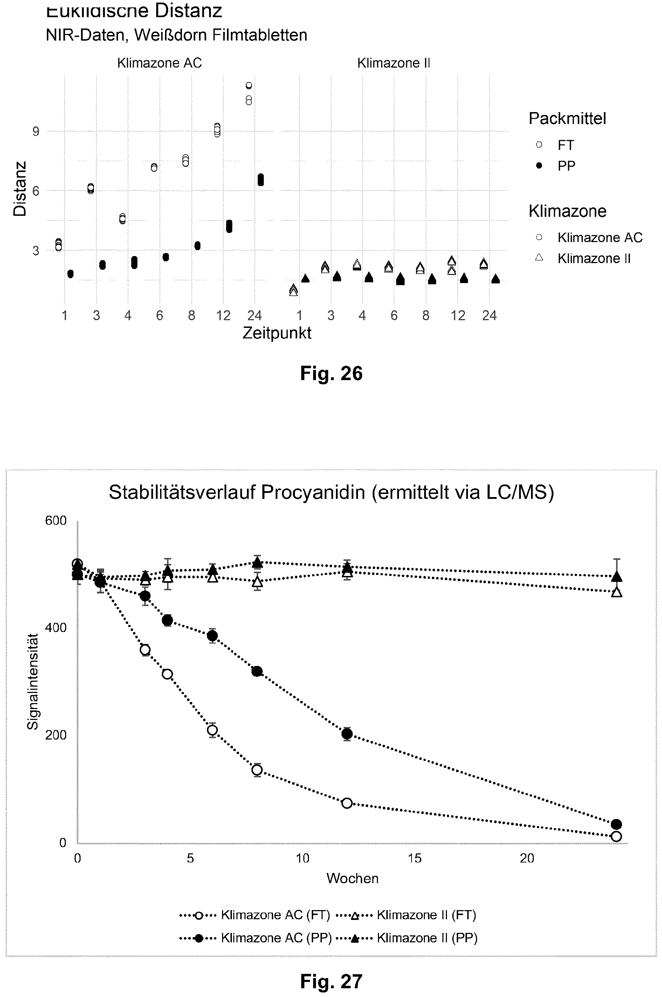

Die Auswertung des NIR-Datensatzes zeigt ein erwartetes Ergebnis. Zum einen nimmt die Distanz unter Klimabedingung AC deutlich schneller zu und erreicht auch größere Maximalwerte im Zeitraum von 24 Wochen. Weiter kann auch klar eine stabilisierende Wirkung des höherwertigen Packmaterials in Form von Blister in Kombination mit einer Faltschachtel (in Abbildungslegende mit "PP" bezeichnet) im Gegensatz zu den Polyethylen-Beuteln (in Abbildungslegende mit "FT" bezeichnet) abgeleitet werden (vgl. Fig. 26). Das gleiche Ergebnis kann auch über eine stellvertretende Markerverbindung (dimeres Procyanidin, m/z 577,14; Retentionszeit = 2,69 min) in einem klassischen Auswerteansatz abgeleitet werden (vgl. Fig. 27). Auch hier sieht man bei den Proben aus Klimazone II eine weitgehende Konstanz und bei Klimazone AC eine deutliche, rasche Degradation. Jedoch ist der klassische Ansatz mit einem wesentlich höherem Mess- und Auswerteaufwand verbunden und ist nicht in der Lage, das entsprechende Produkt in seiner vollen stofflichen Komplexität zu repräsentieren. Das neuartige Verfahren löst beide Einschränkungen, da keine spezifische Selektion und Prozessierung einzelner Rohdatenpunkte durch einen Anwender erforderlich ist, sondern das vollständige Gesamtinhaltsstoffspektrum über die NIR-Spektren erfasst wird und das neue Verfahren garantiert das Verhalten der untersuchten Probe widerspiegelt.

Es versteht sich, dass Aspekte der hier beschriebenen Ausführungsformen, die im Rahmen einer Vorrichtung beschrieben wurden, auch eine Beschreibung eines entsprechenden Verfahrens darstellen. Einige oder alle Verfahrensschritte können durch (oder unter Verwendung) einer Hardwarevorrichtung ausgeführt werden, wie es zum Beispiel ein Prozessor, ein Mikroprozessor, ein programmierbarer Computer oder eine elektronische Schaltung sein kann. In einigen Ausführungsbeispielen können ein oder mehrere der wichtigsten Verfahrensschritte durch eine solche Vorrichtung ausgeführt werden.

Ausführungsbeispiele der Erfindung können in Hardware und/oder Software implementiert werden. Die Implementierung kann mit einem nicht-flüchtigen Speichermedium wie einem digitalen Speichermedium, wie beispielsweise einer Diskette, einer DVD, einem Blu-Ray, einer CD, einem ROM, einem PROM und EPROM, einem EEPROM oder einem FLASH-Speicher, durchgeführt werden, auf dem elektronisch lesbare Steuersignale gespeichert sind, die mit einem programmierbaren Computersystem so zusammenwirken (können), dass das jeweilige Verfahren durchgeführt wird. Daher kann das digitale Speichermedium computerlesbar sein. Einige Ausführungsbeispiele gemäß der Erfindung umfassen einen Datenträger mit elektronisch lesbaren Steuersignalen, die mit einem programmierbaren Computersystem zusammenwirken können, so dass eines der hierin beschriebenen Verfahren durchgeführt wird.

Im Allgemeinen können Ausführungsbeispiele der vorliegenden Erfindung als Computerprogrammprodukt mit einem Programmcode implementiert werden, wobei der Programmcode für die Ausführung eines der Verfahren wirksam ist, wenn das Computerprogrammprodukt auf einem Computer läuft. Der Programmcode kann beispielsweise auf einem maschinenlesbaren Träger gespeichert werden. Weitere Ausführungsbeispiele umfassen das Computerprogramm zur Durchführung eines der hierin beschriebenen Verfahren, das auf einem maschinenlesbaren Träger gespeichert ist.

Ein weiteres Ausführungsbeispiel der vorliegenden Erfindung ist ein Speichermedium (oder ein Datenträger oder ein computerlesbares Medium), das ein darauf gespeichertes Computerprogramm zum Ausführen eines der hierin beschriebenen Verfahren umfasst, wenn es von einem Prozessor ausgeführt wird. Der Datenträger, das digitale Speichermedium oder das aufgezeichnete Medium sind in der Regel greifbar und/oder nicht übergangslos. Eine weiteres Ausführungsbeispiel der vorliegenden Erfindung ist eine Vorrichtung, wie hierin beschrieben, die einen Prozessor und das Speichermedium umfasst.

Ein weiteres Ausführungsbeispiel der Erfindung ist ein Datenstrom oder eine Signalfolge, die das Computerprogramm zur Durchführung eines der hierin beschriebenen Verfahren darstellt. Der Datenstrom oder die Signalfolge kann beispielsweise so konfiguriert werden, dass sie über eine Datenkommunikationsverbindung, beispielsweise über das Internet, übertragen werden.

Ein weiteres Ausführungsbeispiel umfasst ein Verarbeitungsmittel, zum Beispiel einen Computer oder eine programmierbare Logikvorrichtung, das konfiguriert oder angepasst ist, um eines der hierin beschriebenen Verfahren auszuführen.

Ein weiteres Ausführungsbeispiel umfasst einen Computer, auf dem das Computerprogramm zum Ausführen eines der hierin beschriebenen Verfahren installiert ist.

Ein weiteres Ausführungsbeispiel gemäß der Erfindung umfasst eine Vorrichtung oder ein System, das konfiguriert ist, um ein Computerprogramm zum Ausführen eines der hierin beschriebenen Verfahren an einen Empfänger zu übertragen. Der Empfänger kann beispielsweise ein Computer, eine mobile Vorrichtung, eine Speichervorrichtung oder dergleichen sein. Die Vorrichtung oder das System kann beispielsweise einen Dateiserver zum Übertragen des Computerprogramms an den Empfänger umfassen.

**Patentansprüche**

1. Ein Verfahren zur Bestimmung der Stabilität von Stoffen oder Stoffgemischen während einer Produktentwicklung, beispielsweise im Rahmen der Arzneimittelforschung, wobei das Verfahren folgende Schritte umfasst:

   - einen computerimplementierten Datenerfassungsschritt (102), in dem ein Computerprogramm Roh- und/oder Meta-Daten für mehrere Produktkandidaten mit jeweils einem Startwert-Messdatensatz und zumindest einem weiteren Messdatensatz empfängt, wobei der Startwert-Messdatensatz und jeder weitere Messdatensatz jeweils ein chemisches, insbesondere phytochemisches, Profil eines jeweiligen Stoffs oder Stoffgemischs des jeweiligen Produktkandidaten repräsentiert;
   - einen computerimplementierten Datenauswertungsschritt (106), der für jeden weiteren Messdatensatz umfasst:

     - Bestimmen (112), durch das Computerprogramm, des Startwert-Messdatensatzes des jeweiligen Stoffs oder Stoffgemischs auf Basis von Roh- und/oder Meta-Daten des Startwert-Messdatensatzes; und
     - Quantifizieren (114), durch das Computerprogramm, der Veränderung des jeweiligen Stoffs oder Stoffgemischs in einem zeitlichen Verlauf in Bezug auf den Startwert-Messdatensatz und den mindestens einen weiteren Messdatensatz mittels eines mathematischen Distanzmaßes, wobei das mathematische Distanzmaß durch einen Benutzer auswählbar ist und wobei der Benutzer für die Quantifizierung der Veränderung des jeweiligen Stoffs oder Stoffgemischs mehrere mathematische Distanzmaße wählen kann, insbesondere iterativ nacheinander, wobei die Quantifizierung auf Basis jedes gewählten mathematischen Distanzmaßes erfolgt; und
     - Durchführen (110), durch das Computerprogramm, einer zusätzlichen qualitativen Analyse, insbesondere einer Hauptkomponentenanalyse, für den Messdatensatz, welche nicht in die Quantifizierung der Veränderung mittels des mathematischen Distanzmaßes eingeht; und

   - einen computerimplementierten Datenausgabeschritt (108), in dem das Computerprogramm für jeden weiteren Messdatensatz die Veränderung des jeweiligen Stoffs oder Stoffgemischs grafisch darstellt, wobei der Datenausgabeschritt (108) umfasst:

     - Darstellen der Veränderung des jeweiligen Stoffs oder Stoffgemischs als Box-Plot; und
     - Darstellen des Ergebnisses der zusätzlichen qualitativen Analyse;

   wobei das Verfahren ferner umfasst:

     - Selektieren einer reduzierten Auswahl von Produktkandidaten, welche die geringste Veränderung zeigen und somit als am stabilsten angenommen werden können; und
     - weiteres Untersuchen nur der reduzierten Auswahl von Produktkandidaten durch weitere dafür spezifische analytische Methoden.

2. Das Verfahren nach Anspruch 1, wobei der Datenauswertungsschritt (106) das Verwenden eines maschinellen Lernmodells umfasst, welches vorzugsweise durch unüberwachtes und/oder überwachtes maschinelles Lernen erzeugt und/oder trainiert wurde.

3. Das Verfahren nach Anspruch 1 oder 2, wobei der zumindest eine Messdatensatz mittels Nahinfrarotspektroskopie, NIR, erhaltene Daten umfasst.

4. Das Verfahren nach irgendeinem der vorhergehenden Ansprüche, wobei der zumindest eine Messdatensatz umfasst:

- mittels UV/VIS-Spektroskopie erhaltene Daten;
- mittels Raman-Spektroskopie erhaltene Daten;
- einen (U)HPLC-Fingerprint;
- einen GC-Fingerprint;
- eine Peaktable aus einem chromatographischen Verfahren; und/oder
- zumindest einen physikalischen, biologischen oder chemischen Parameter, insbesondere Zuckergehalt, Zerfallsgeschwindigkeit, Farbe, Bruchfestigkeit, Zerfallszeit, Friabilität, Dichte, Viskosität, Brechungsindex und/oder optischer Drehwinkel.

5. Das Verfahren nach irgendeinem der vorhergehenden Ansprüche, wobei die Quantifizierung der Veränderung des jeweiligen Stoffs oder Stoffgemischs auf der Gesamtheit der Inhaltsstoffe des jeweiligen Stoffs oder Stoffgemischs basiert.

6. Das Verfahren nach irgendeinem der vorhergehenden Ansprüche, wobei das mathematische Distanzmaß ausgewählt ist aus der folgenden Gruppe: euklidische Distanz, Mahalanobis-Distanz, Manhattan-Distanz, Pearson-Distanz und/oder Gower-Distanz.

7. Das Verfahren nach irgendeinem der vorhergehenden Ansprüche, ferner umfassend:
einen Datenvorverarbeitungsschritt (104), umfassend:

- Durchführen einer Streulichtkorrektur des zumindest einen Messdatensatzes, insbesondere wenn dieser Nahinfrarot-Messdaten umfasst;
- Durchführen einer Zentrierung, Normierung und/oder Skalierung des zumindest einen Messdatensatzes; und/oder
- Durchführen einer Hauptkomponentenanalyse.

8. Das Verfahren nach irgendeinem der vorhergehenden Ansprüche, wobei der Datenausgabeschritt (108) umfasst:

- Darstellen von Mittelwerten, Medianen, 0,25-/0,75-Quantilen, Streuungsmaßen, Hervorheben möglicher Ausreißer-Kandidaten; und/oder
- Durchführen zumindest eines statistischen Tests, insbesondere t-Test, Wilcoxon-Rang-Summen-Test, One-Way-ANOVA und/oder Kruskall-Wallis-Test.

9. Das Verfahren nach irgendeinem der vorhergehenden Ansprüche, wobei der Stoff oder das Stoffgemisch feste und/oder flüssige und/oder gasförmige Stoffe umfasst.

10. Das Verfahren nach irgendeinem der vorhergehenden Ansprüche, wobei der Stoff oder das Stoffgemisch biologische, chemische, pflanzliche, tierische, humane Stoffe oder Stoffgemische, pharmazeutische Zusammensetzungen, pflanzliche Arzneimittel, chemische und/oder biologische Arzneimittel, Zellen, Zelltherapeutika (beispielweise Gentherapeutika, z.B. CAR T-Zellen (Chimeric Antigen Receptor T-Zellen), NK-Zellen (Natural Killer-Zellen) somatische Zelltherapeutika, biotechnologisch bearbeitete Gewebeprodukte/tissue engineered products, Gewebe, Stammzellen, Stammzellprodukte bzw. -zubereitungen, z.B. CD34+-Zellen, CD19+-Zellen, CD20+-Zellen, HEK295-Zellen, TCR alpha/beta-Zellen, TCR gamma/delta-Zellen, CD3+-, CD4+-, CD8+- CD133+-Zellen), Blut, Blutprodukte, Organe, Arzneitees, Extrakte, insbesondere Eisenkrautextrakt, Thymian, Rosmarin und Kamillen-Arzneidrogenmischungen, z.B. als ethanolischer Auszug oder in Pulverform, Tropfen, Tabletten, Dragees, Kapseln, Pulver, Granulate, Lösungen, Suspensionen, Säfte, Nahrungsmittel, insbesondere Fleisch oder Hackfleisch, Fruchtsaft, insbesondere Orangensaft, Nahrungsergänzungsmittel, Kosmetika, Emulsionen, Salben und/oder Cremes umfasst, sowie Verpackungen, Packmittel, Folien, insbesondere Polyethylen, Polyvinylchlorid, umfasst.

11. Ein Computerprogramm, umfassend Befehle, die bei der Ausführung des Programms durch einen Computer diesen veranlassen, die computerimplementierten Schritte des Verfahrens nach irgendeinem der Ansprüche 1-10 auszuführen.

12. Eine Vorrichtung, insbesondere ein Messinstrument oder ein Server-Computer, umfassend Mittel zur Ausführung der computerimplementierten Schritte des Verfahrens nach irgendeinem der Ansprüche 1-10.

**Claims**

1. A method for determining the stability of substances or substance mixtures during product development, for example in the context of drug research, the method comprising the following steps:

   - a computer-implemented data acquisition step (102) in which a computer program receives raw and/or meta data for a plurality of product candidates, each having a starting value measurement data set and at least one further measurement data set, the starting value measurement data set and each further measurement data set each representing a chemical, in particular phytochemical, profile of a respective substance or substance mixture of the respective product candidate;
   - a computer-implemented data evaluation step (106), which comprises for each further measurement data set:

      - determining (112), by the computer program, the starting value measurement data set of the respective substance or substance mixture on the basis of raw and/or meta data of the starting value measurement data set; and
      - quantifying (114), by the computer program, the change in the respective substance or substance mixture over time with respect to the initial value measurement data set and the at least one further measurement data set by means of a mathematical distance measure, wherein the mathematical distance measure can be selected by a user and wherein the user can select several mathematical distance measures for the quantification of the change in the respective substance or substance mixture, in particular iteratively one after the other, wherein the quantification is carried out on the basis of each selected mathematical distance measure; and
      - performing (110), by the computer program, an additional qualitative analysis, in particular a principal component analysis, for the measurement data set, which is not included in the quantification of the change by means of the mathematical distance measure; and

   - a computer-implemented data output step (108) in which the computer program graphically represents the change in the respective substance or substance mixture for each further set of measurement data, wherein the data output step (108) comprises:

      - displaying the change in the respective substance or substance mixture as a box plot; and
      - displaying the result of the additional qualitative analysis;

   wherein the method further comprises:

      - selecting a reduced range of product candidates that show the least change and can therefore be assumed to be the most stable; and
      - further investigation only of the reduced selection of product candidates using other specific analytical methods

2. The method according to claim 1, wherein the data evaluation step (106) comprises using a machine learning model, preferably generated and/or trained by unsupervised and/or supervised machine learning.

3. The method according to claim 1 or 2, wherein the at least one set of measurement data comprises data obtained by near infrared spectroscopy, NIR.

4. The method according to any one of the preceding claims, wherein the at least one set of measurement data comprises:

   - data obtained by means of UV/VIS spectroscopy;
   - data obtained using Raman spectroscopy;
   - a (U)HPLC fingerprint;
   - a GC fingerprint
   - a peaktable from a chromatographic process; and/or
   at least one physical, biological or chemical parameter, in particular sugar content, disintegration rate, color, breaking strength, disintegration time, friability, density, viscosity, refractive index and/or optical angle of rotation.

5. The method according to any one of the preceding claims, wherein the quantification of the change in the respective substance or mixture of substances is based on the totality of the ingredients of the respective substance or mixture of substances.

6. The method according to any one of the preceding claims, wherein the mathematical distance measure is selected from the following group: Euclidean distance, Mahalanobis distance, Manhattan distance, Pearson distance and/or Gower distance.

7. The method according to any one of the preceding claims, further comprising:
   a data pre-processing step (104), comprising:

   - performing a stray light correction of the at least one measurement data set, in particular if this comprises near-infrared measurement data;
   - performing centering, normalization and/or scaling of the at least one measurement data set; and/or
   - performing a principal component analysis.

8. The method according to any one of the preceding claims, wherein the data output step (108) comprises:

   - displaying mean values, medians, 0.25/0.75 quantiles, measures of dispersion, highlighting of possible outlier candidates; and/or
   - performing at least one statistical test, in particular t-test, Wilcoxon rank-sum test, one-way ANOVA and/or Kruskall-Wallis test.

9. The method according to any one of the preceding claims, wherein the substance or mixture of substances comprises solid and/or liquid and/or gaseous substances.

10. The method according to any of the preceding claims, wherein the substance or mixture of substances comprises biological, chemical, herbal, animal, human substances or mixtures of substances, pharmaceutical compositions, herbal medicinal products, chemical and/or biological medicinal products, cells, cell therapeutics (for example gene therapeutics, e.g. CAR T cells (Chimeric Antigen Receptor T cells), NK cells (Natural Killer cells), somatic cell therapeutics, bioengineered tissue products/engineered tissue products).e.g. CAR T cells (Chimeric Antigen Receptor T cells), NK cells (Natural Killer cells) somatic cell therapeutics, biotechnologically processed tissue products/tissue engineered products, tissue, stem cells, stem cell products or preparations, e.g. CD34+ cells, CD19+ cells, CD20+ cells, HEK295 cells, TCR alpha/beta cells, TCR gamma/delta cells, CD3+, CD4+, CD8+, CD133+ cells), blood, blood products, organs, medicinal teas, extracts, in particular verbena extract, thyme, rosemary and chamomile medicinal drug mixtures, e.g. as an ethanolic extract or in powder form, drops, tablets, coated tablets, capsules, powders, granules, solutions, suspensions, juices, foodstuffs, in particular meat or minced meat, fruit juice, in particular orange juice, food supplements, cosmetics, emulsions, ointments and/or creams, as well as packaging, packaging materials, films, in particular polyethylene, polyvinyl chloride.

11. A computer program comprising instructions which, when the program is executed by a computer, cause the computer to perform the computer-implemented steps of the method of any one of claims 1-10.

12. An apparatus, in particular a measuring instrument or a server computer, comprising means for carrying out the computer-implemented steps of the method according to any one of claims 1-10.

**Revendications**

1. Un procédé pour déterminer la stabilité de substances ou de mélanges de substances pendant le développement d'un produit, par exemple dans le cadre de la recherche de médicaments, le procédé comprenant les étapes suivantes:

   - une étape d'acquisition de données (102) mise en oeuvre par ordinateur, dans laquelle un programme informatique reçoit des données brutes et/ou des métadonnées pour plusieurs produits candidats ayant chacun un ensemble de données de mesure de valeur de départ et au moins un autre ensemble de données de mesure, l'ensemble de données de mesure de valeur de départ et chaque autre ensemble de données de mesure représentant chacun un profil chimique, en particulier phytochimique, d'une substance ou d'un mélange de substances respectif du produit candidat respectif ;

- une étape d'évaluation de données (106) mise en oeuvre par ordinateur, comprenant pour chaque autre ensemble de données de mesure :

- déterminer (112), par le programme informatique, l'ensemble de données de mesure de valeur initiale de la substance ou du mélange de substances respectif sur la base des données brutes et/ou méta de l'ensemble de données de mesure de valeur initiale ; et

- quantifier (114), par le programme informatique, la variation de la substance ou du mélange de substances respectif dans une évolution temporelle par rapport à l'ensemble de données de mesure de valeur de départ et à l'au moins un autre ensemble de données de mesure au moyen d'une mesure de distance mathématiquela mesure de distance mathématique pouvant être sélectionnée par un utilisateur et l'utilisateur pouvant sélectionner plusieurs mesures de distance mathématiques pour la quantification de la variation de la substance ou du mélange de substances respectif, en particulier de manière itérative l'une après l'autre, la quantification s'effectuant sur la base de chaque mesure de distance mathématique sélectionnée ; et

- effectuer (110), par le programme informatique, une analyse qualitative supplémentaire, en particulier une analyse en composantes principales, pour l'ensemble de données de mesure, qui n'entre pas dans la quantification du changement au moyen de la mesure mathématique de distance ; et

- une étape de sortie de données (108) mise en oeuvre par ordinateur, dans laquelle le programme informatique représente graphiquement, pour chaque autre ensemble de données de mesure, la variation de la substance ou du mélange de substances respectif, l'étape de sortie de données (108) comprenant :

- présenter la modification de la substance ou du mélange de substances concerné sous forme de box plot ; et
- présenter le résultat de l'analyse qualitative supplémentaire ;

le procédé comprenant en outre

- sélectionner une sélection réduite de produits candidats qui présentent le moins de changements et qui peuvent donc être considérés comme les plus stables ; et
- poursuite de l'examen uniquement de la sélection réduite de produits candidats par d'autres méthodes analytiques spécifiques à cet effet

2. Le procédé selon la revendication 1, dans lequel l'étape d'évaluation des données (106) comprend l'utilisation d'un modèle d'apprentissage automatique qui a été généré et/ou entraîné, de préférence par un apprentissage automatique non supervisé et/ou supervisé.

3. Le procédé selon la revendication 1 ou 2, dans lequel ledit au moins un ensemble de données de mesure comprend des données obtenues par spectroscopie dans le proche infrarouge, NIR.

4. Le procédé selon l'une quelconque des revendications précédentes, dans lequel ledit au moins un ensemble de données de mesure comprend :

- données obtenues par spectroscopie UV/VIS ;
- données obtenues par spectroscopie Raman ;
- une empreinte digitale (U)HPLC ;
- une empreinte GC
- une pectable issue d'un procédé chromatographique ; et/ou
au moins un paramètre physique, biologique ou chimique, notamment la teneur en sucre, la vitesse de désintégration, la couleur, la résistance à la rupture, le temps de désintégration, la friabilité, la densité, la viscosité, l'indice de réfraction et/ou l'angle de rotation optique.

5. Le procédé selon l'une quelconque des revendications précédentes, dans lequel la quantification de la modification de la substance ou du mélange de substances considéré est basée sur l'ensemble des composants de la substance ou du mélange de substances considéré.

6. Le procédé selon l'une quelconque des revendications précédentes, dans lequel la mesure mathématique de distance est choisie dans le groupe suivant : distance euclidienne, distance de Mahalanobis, distance de Manhattan, distance de Pearson et/ou distance de Gower.

7. Le procédé selon l'une quelconque des revendications précédentes, comprenant en outre :
une étape de prétraitement de données (104), comprenant :

- effectuer une correction de la lumière parasite du au moins un ensemble de données de mesure, en particulier si celui-ci comprend des données de mesure dans le proche infrarouge ;
- effectuer un centrage, une normalisation et/ou une mise à l'échelle de l'au moins un ensemble de données de mesure ; et/ou
- Effectuer une analyse en composantes principales.

8. Le procédé selon l'une quelconque des revendications précédentes, dans lequel l'étape de sortie de données (108) comprend :

- Représenter les moyennes, les médianes, les quantiles 0,25/0,75, les mesures de dispersion, mettre en évidence les candidats possibles aux valeurs aberrantes ; et/ou
- effectuer au moins un test statistique, en particulier le test t, le test de la somme des rangs de Wilcoxon, l'ANOVA à sens unique et/ou le test de Kruskall-Wallis.

9. Le procédé selon l'une quelconque des revendications précédentes, dans lequel la substance ou le mélange de substances comprend des substances solides et/ou liquides et/ou gazeuses.

10. Procédé selon l'une quelconque des revendications précédentes, dans lequel la substance ou le mélange de substances est une substance ou un mélange de substances biologiques, chimiques, végétales, animales, humaines, des compositions pharmaceutiques, des médicaments à base de plantes, des médicaments chimiques et/ou biologiques, des cellules, des produits de thérapie cellulaire (par exemple des produits de thérapie génique, par ex.par ex. cellules CAR T (cellules T réceptrices d'antigènes chimériques), cellules NK (cellules Natural Killer), produits de thérapie cellulaire somatique, produits tissulaires issus de la biotechnologie/tissue engineered products, tissus, cellules souches, produits ou préparations de cellules souches, par ex. cellules CD34+, cellules CD19+, cellules CD20+, cellules HEK295, cellules TCR alpha/bêta, cellules TCR gamma/delta, cellules CD3+, CD4+, CD8+-CD133+), sang, produits sanguins, organes, tisanes médicinales, extraits, en particulier extrait de verveine, thym, romarin et mélanges d'extraits médicinaux de camomille, par ex. sous forme d'extrait éthanolique ou de poudre, des gouttes, des comprimés, des dragées, des capsules, des poudres, des granulés, des solutions, des suspensions, des jus, des aliments, en particulier de la viande ou de la viande hachée, du jus de fruit, en particulier du jus d'orange, des compléments alimentaires, des produits cosmétiques, des émulsions, des pommades et/ou des crèmes, ainsi que des emballages, des moyens d'emballage, des films, en particulier du polyéthylène, du chlorure de polyvinyle.

11. Programme d'ordinateur comprenant des instructions qui, lorsque le programme est exécuté par un ordinateur, amènent ce dernier à exécuter les étapes, mises en œuvre par ordinateur, du procédé selon l'une quelconque des revendications 1 à 10.

12. Un dispositif, en particulier un instrument de mesure ou un ordinateur serveur, comprenant des moyens pour exécuter les étapes mises en œuvre par ordinateur du procédé selon l'une quelconque des revendications 1 à 10.

**Fig. 1**

Rohdaten mit entfernter Detektorsättigung

**Fig. 3**

Rohdaten

Rohdaten nach Streulichtkorrektur

Fig. 4

## Boxplot euklidischer Distanzen vom Mittelwert

**Fig. 5**

## NIR-Auswertung: Hackfleisch
### Distanzmittelwerte, Fehlerbalken: Standardfehler

**Fig. 6**

|  | **40 °C** | **RT** |
|---|---|---|
| **Start Beobachtungs-zeitraum:** | | |
| **Ende Beobachtungs-zeitraum:** | (1) | (3) (2) |

**Fig. 7**

**Boxplot euklidischer Distanzen vom Mittelwert**

**Fig. 8**

# Kinetik Extraktion / Onlinemonitoring
## Fehlerbalken: SEM

```
Formula: Distanz ~ (Distanz_max * t)/(k + t)

Parameters:
            Estimate Std. Error t value Pr(>|t|)
Distanz_max 0.144217   0.006329  22.788  < 2e-16 ***
k           3.949141   0.976682   4.043 0.000175 ***
---
Signif. codes:  0 '***' 0.001 '**' 0.01 '*' 0.05 '.' 0.1 ' ' 1
```

**Fig. 9**

# Kinetik Zellversuch / Kontrolle
## Fehlerbalken: SEM

```
Formula: Distanz ~ (Distanz_max * t)/(k + t)

Parameters:
            Estimate Std. Error t value Pr(>|t|)
Distanz_max 0.125087   0.005092  24.565 5.83e-11 ***
k           2.341276   1.177280   1.989   0.0722 .
---
Signif. codes:  0 '***' 0.001 '**' 0.01 '*' 0.05 '.' 0.1 ' ' 1
```

## Kinetik Zellversuch / Ethanol 3%
Fehlerbalken: SEM

Formula: Distanz ~ (Distanz_max * t)/(k + t)

Parameters:
```
          Estimate Std. Error t value Pr(>|t|)
Distanz_max  0.19428    0.01468  13.233 4.23e-08 ***
k           12.93688    7.55989   1.711    0.115
---
Signif. codes:  0 '***' 0.001 '**' 0.01 '*' 0.05 '.' 0.1 ' ' 1
```

**Fig. 11**

## Kinetik Zellversuch / Ethanol 6%
Fehlerbalken: SEM

Formula: Distanz ~ (Distanz_max * t)/(k + t)

Parameters:
```
          Estimate Std. Error t value Pr(>|t|)
Distanz_max  0.27191    0.01491  18.242 1.43e-09 ***
k           11.37824    5.19238   2.191   0.0508 .
---
Signif. codes:  0 '***' 0.001 '**' 0.01 '*' 0.05 '.' 0.1 ' ' 1
```

Fig. 13

Fig. 14

## Boxplot euklidische Distanzen Arzneitee

**Fig 15**

## Vergleich Stabilitäten/Wiederfindung Arzneitee
### NIR/Stabilitätsalgorithmus vs. HPLC Referenzmessung

**Fig 16**

LC/MS-Rohdaten

20% EtOH Auszug (Rosmarin, Thymian, Kamille)

**Fig. 17**

Euklidische Distanz

LC/MS-Daten, 20% EtOH Auszug (Rosmarin, Thymian, Kamille)

**Fig. 18**

Kinetik
20% EtOH Auszug (Rosmarin, Thymian, Kamille)

Distanz / Zeit

Klimazone
AC
KII

**Fig. 19**

Stabilitätsverlauf Analyt in
20%-igem ethanolisch wässrigem Auszug

Signalintensität / Wochen

···●··· Klimazone AC    ····▲··· Klimazone II

**Fig. 20**

Euklidische Distanz

NIR-Daten, Packmittelvergleich

**Fig. 21**

**Fig. 22**

EP 4 515 551 B1

**Fig. 23**

Stabilitätsverlauf Analyt in Arzneidrogen-Pulvermischung

**Fig. 24**

**Fig.25**

## Euklidische Distanz
### NIR-Daten, Weißdorn Filmtabletten

**Fig. 26**

**Fig. 27**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **VON LEI FENG et al.** Nondestructive Detection of Postharvest Quality of Cherry Tomatoes Using a Portable NIR Spectrometer and Chemometric Algorithms. *Food Analytical Methods*, 2019